# EUROPÄISCHE PATENTSCHRIFT

(11) **EP 1 226 204 B1**
(45) Veröffentlichungstag und Bekanntmachung des Hinweises auf die Patenterteilung: **11.01.2006**
(21) Anmeldenummer: 00956162.2
(22) Anmeldetag: 04.07.2000
(51) Int. Cl.: C08G 81/00, C12N 15/87

(54) **KATIONISCHE BLOCKCOPOLYMERE**
CATIONIC BLOCK COPOLYMERS
COPOLYMERES SEQUENCES CATIONIQUES

(30) Priorität: 15.07.1999 DE 19933024
(43) Veröffentlichungstag der Anmeldung: 31.07.2002
(73) Patentinhaber: MedInnova Gesellschaft für medizinische Innovationen aus akademischer Forschung mbH, 35037 Marburg (DE)
(72) Erfinder: KISSEL, Thomas, 79219 Staufen (DE); PETERSEN, Holger, 35037 Marburg (DE); FISCHER, Dagmar, 35037 Marburg (DE); KUNATH, Klaus, 35041 Marburg (DE); VON HARPE, Anke, 35041 Marburg (DE)
(74) Vertreter: Olbricht, Karl Heinrich
(86) Internationale Anmeldenummer: PCT/EP2000/006214
(87) Internationale Veröffentlichungsnummer: WO 2001/005875

(56) Entgegenhaltungen:
- WO-A-92/07023
- WO-A-98/59064
- DE-A- 3 901 281
- US-A- 5 204 196
- SIDOROV S. N. ET AL: "Stabilization of Metal Nanoparticles in Aqueous Medium by Polyethyleneoxide-Polyethyleneimine Block Copolymers" JOURNAL OF COLLOID AND ITERFACE SCIENCE, Bd. 212, Nr. 2, 1999, Seiten 197-211, XP002154207

## Beschreibung

Aus der WO98/59064 sind PEI-PEG-Blockcopolymere und deren Verwendung als Vehikel für den Transport von Nukleinsäure in höhere eukaryotische Zellen bekannt. Das beschriebene Copolymer wurde aus verzweigten PEI und linearen PEG aufgebaut. Für die PEGylierung des PEI wurde Methoxy-succinimidyl-propionat-PEG verwendet.

S. V. Vinogradov, T. K. Bronich und A. V. Kabanov (Bioconjugate Chem. 1998, 9, 805-812) beschreiben die Darstellung von PEI-PEG- und Polyspermin-PEG-Blockcopolymere durch Verwendung von verzweigtem PEI und verzweigtem Polyspermine durch Kopplungsreaktion mit einem mit 1,1 '-Carbonyldiimidazol aktivierten MonomethoxyPEG. Die Copolymere wurden für die Komplexierung mit Oligonukleotiden verwendet.

In L. M. Bronstein, M. Antonietti, et. al. (inorganica Chimica Acta 1998, 280, 348-354) werden PEI-PEG-Blockcopolymere und deren Herstellung durch Kopplung von verzweigtem PEI mit MonomethoxyPEG, das über eine endständige Säurechloridfunktion verfügt, und deren Verwendung zur Darstellung von Metallkolloiden beschrieben.

V. Toncheva et al. (Biochimica et Biophysika Acta 1998, 138, 354 - 358) betrifft Blockcopolymere, bestehend aus Poly(L-lysin) und mehreren hydrophilen Polymeren, wie PEG, Dextran und Poly(N-(2-hydroxypropyl)methacrylamid, Verfahren zu deren Herstellung und deren Verwendung als Vehikel für den Gentransfer von Nukleinsäure.

Diese bekannten Blockcopolymere haben folgende drei Punkte gemeinsam:
1. Das kationische Polymer ist mit Seitenarmen eines hydrophilen, nichtionischen Polymers versehen.
2. In allen Fällen wurde dazu der reaktive Terminus des hydrophilen, nichtionischen Polymers für die Kopplungsreaktion mit dem kationischen Polymer durch ein Reagenz aktiviert, das im erzeugtem Copolymer ein Brückenglied zwischen den Blöcken darstellt.
3. Das hydrophile, nichtionische Polymer war in allen Fällen ein lineares Polymer.

Weiterhin sind aus US-A 5 204 196 Polymere bekannt, die ein PEI-Rückgrat enthalten, an das mittels eines Isocyanats ein Polyethylenglykolether der Formel -O(CH₂-CH₂)ₙ-CH₃ gebunden ist. Diese Polymere werden in Kombination mit anorganischen Salzen als feste elektrische Leiter verwendet.

Es wurden neue kationische Blockcopolymere der allgemeinen Formel I und II

(I) A(-X-B)ₙ

(II) C(-Y-D)ₘ

gefunden, worin
- A: für ein hydrophiles, nichtionisches, lineares oder verzweigtes Polymer mit einem Molekulargewicht von 100 bis 10.000.000 g/mol, vorzugsweise von 1.000 bis 100.000 g/mol und insbesondere von 5.000 bis 50.000 g/mol steht;
- B: für ein lineares oder verzweigtes Polyethylenimin (PEI) mit einem Molekulargewicht von 100 bis 1.000.000 g/mol, vorzugsweise von 400 bis 100.000 g/mol und insbesondere von 400 bis 50.000 g/mol steht;
- X: für eine direkte Bindung der Blöcke A und B oder für ein Brückenglied mit folgenden Strukturen steht, dessen C-teminale Seite mit einem Stickstoffatom des PEIs verknüpft ist:
-OC(O)NH(CH₂)ₒNHC(O)- mit o = 1 bis 20, vorzugsweise 2 bis 10, insbesondere 4 bis 6,
-OC(O)NH(Aryl)NHC(O)- mit Aryl = aromatische Einheit mit vorzugsweise 6 - 14 C-Atomen bestehend aus einem oder mehreren aromatischen Kernen, die in kondensierter oder in polyphenylischer Form miteinander verbunden sind, vorzugsweise mit einem Kern, insbesondere Toluyl-, -O(CH₂)ₚC(O)- mit p = 1 bis 10, vorzugsweise 1 bis 3, insbesondere 1,
-OCH₂CH(OH)CH₂-,
-OC(O)-, oder
-O(CH₂)_{q}- mit q = 1 bis 20, vorzugsweise 1 bis 6, insbesondere 1 bis 3;
- n: für eine ganze Zahl von
1) 1 bis 200, vorzugsweise,
2) 1 bis 50,
3) 1 bis 12,
4) 1 bis 8 oder, besonders bevorzugt,
5) 2 bis 8 steht;
- C: für ein lineares oder verzweigtes PEI mit einem Molekulargewicht von 100 bis 1.000.000 g/mol, vorzugsweise von 400 bis 100.000 g/mol und insbesondere von 400 bis 50.000 g/mol steht;
- D: für einen Rest eines über O gebundenen Polyethylenglykols der Formel

-(CH₂CH₂O)ₙ-R¹

steht, worin n' = 3 bis 25.000, vorzugsweise 10 bis 5.000 und insbesondere 10 bis 1.000 ist und R¹ für Wasserstoff, einen aliphatischen Rest wie (C₁-C₆)-Alkyl (Methyl, Ethyl, tert.-Butyl und dergleichen) oder eine andere OH-Schutzgruppe wie Acyl (z.B. ggf. substituiertes Benzoxycarbonyl), ggf. substituiertes Benzyl, Picolyl, oder einen zellulären Liganden, um die spezifische Aufnahme eines Nukleinsäure-Copolymer-Komplexes durch Bindung an Zelloberflächenproteine, insbesondere Rezeptoren, zu bewirken, steht;
- Y: für eine direkte Bindung der Blöcke C und D oder für ein Brückenglied mit folgenden Strukturen steht, dessen C-teminale Seite mit einem Stickstoffatom des PEIs verknüpft ist:
-C(O)NH(Aryl)NHC(O)O- mit Aryl = aromatische Einheit mit vorzugsweise 6 bis 14 C-Atomen bestehend aus einem oder mehreren aromatischen Kernen, die in kondensierter oder in polyphenylischer Form miteinander verbunden sind, vorzugsweise mit einem Kern, insbesondere Toluyl-,
-(CH₂)ₜC(O)O- mit t = 2 bis 10, vorzugsweise 2 bis 3, insbesondere 2,
-CH₂CH(OH)CH₂O-, oder
-(CH₂)ᵤO- mit u = 1 bis 20, vorzugsweise 1 bis 6, insbesondere 1 bis 3;

und
- m: für eine ganze Zahl von
1) 1 bis 200, vorzugsweise
2) 1 bis 100, insbesondere
3) 1 bis 50 steht,

mit der Maßgabe, daß die Reste und Variablen in Formel II so definiert sind, daß keine Verbindungen der Formel I davon umfaßt werden.

Die erfindungsgemäßen kationischen Blockcopolymeren unterscheiden sich von den bekannten Blockcopolymeren in mindestens einem der drei folgenden Merkmalen:

Ein hydrophiles, nichtionisches Polymer ist mit Seitenarmen eines kationischen Polymers versehen.
1. Die Brückenglieder unterscheiden sich von jenen bekannter Blockcopolymere.
2. Sie weisen ein verzweigtes hydrophiles, nichtionisches Polymer auf.

Unter A werden vorzugsweise aus Kohlenstoff und Sauerstoff aufgebaute lineare oder verzweigte Polymere, die gegebenenfalls auch cyclische, sternförmige oder dendritische Strukturen enthalten können, wie beispielsweise Reste von linearem PEG, mehrarmig verzweigten PEG, sternförmige PEG, Polysaccharide einschließlich Cyclodextrine, PVA, Arborole (Dendrimere mit endständigen Hydroxygruppen), vorzugsweise aber lineare und mehrarmig verzweigte und sternförmige PEGs verstanden. Letztere sind kommerziell u. a. von Aldrich, Fluka, Sigma und Shearwater erhältlich.

Unter B und C werden lineare oder verzweigte Polyethylenimin-Reste verstanden, welche die Formel III

(III) -[CH₂CH₂N^{(z+)}(R²)ₓ]_{y}-H [A⁻]_{w}

aufweisen,
in welchen R² für gleiche oder verschiedene Reste steht und Wasserstoff oder einen Rest der Formel IV bedeutet,

(IV) -[CH₂CH₂N^{(z'+)}(R³)_{x'}]_{y'}-H [A⁻]_{w'}

- R³: für gleiche oder verschiedene Reste steht, die (rekursiv) wie R² definiert sind,
- A⁻: für ein Äquivalent eines geeigneten, vorzugsweise anorganischen Anions stehen, wie OH⁻, Cl⁻, Br⁻ und dergleichen,

x und x' gleich oder verschieden sind und 1 oder 2 bedeuten,
y und y' gleich oder verschieden sind und für ganze Zahlen stehen, die so gewählt werden, daß die Reste B bzw. C ein anteiliges Molekulargewicht von 100 bis 1.000.000 g/mol, vorzugsweise von 400 bis 100.000 g/mol und insbesondere von 400 bis 50.000 g/mol aufweisen, wobei y' auch 0 sein kann,
z und z' gleich oder verschieden, z = x-1 und z' = x'-1 sind,
und
w und w' gleiche oder verschiedene ganze Zahlen sind, die so gewählt werden, daß die positiven Ladungen in den Resten der Formeln III und IV ausgeglichen werden.

Die Polyethylenimine können in an sich bekannter Weise hergestellt werden oder sind unter den BASF-Markennamen Lupasol® oder unter der Bezeichnung Polyethylenimin oder Ethylenimin Polymer in verschiedenen Molekulargewichten von 400 bis 2.000.000 g/mol im Handel (von Aldrich, Sigma, Fluka oder direkt von der BASF) erhältlich. Bevorzugt sind für B Polyethylenimine mit einem Molekulargewicht von 400 bis 2.000 g/mol und für C Polyethylenimine mit einem Molekulargewicht von 400 bis 800.000 g/mol, besonders bevorzugt von 400 bis 25.000 g/mol.

Die unter D beschriebenen Gruppen sind Reste von Polyethylenglykolen, die an einem Terminus durch einen Rest R¹ wie beispielsweise Methyl oder eine andere geeignete Schutzgruppe geschützt sind. R¹ kann aber auch eine Gruppe sein, die eine spezifische oder unspezifische biologische Funktion ausübt, insbesondere ein Ligand für Wechselwirkungen mit Rezeptoren zur zielzell-spezifischen Aufnahme eines Blockcopolymer-Wirkstoff-Komplexes in höhere eukaryotische Zellen und deren Zellkern, wobei der Wirkstoff vorzugsweise ein Oligonukleotid oder ein Gen darstellt (Gentargeting). R¹ kann somit auch ein Ligand für eine spezifische Wechselwirkung und Aufnahme in Zielorgan-gewebe oder -zellen, beispielsweise Proteine, insbesondere Antikörper oder Antikörperfragmente wie Fab, F(ab)₂, scFv, Cyto- oder Lymphokine, wie Interleukine (IL-2 bis x), Interferon GM-CSF, Wachstumsfaktoren, wie EGF, PDGF, FGF, EPO,
Integrine wie ICAM, VCAM oder
Glykoproteine wie Lektine oder glykosilierte Proteine (s. o.) oder
Lipoproteine wie LDL, HDL oder
Transporterproteine wie Transferrin oder
Peptide wie LH-RH, Calcitonin, Oxytocin, Insulin, Somatostatin, IGF, RGD oder
Kohlenhydrate wie Galactose, Mannose, Glucose, Lactose oder
Hormone wie Steroide, THR oder
Vitamine wie B₁₂, Folsäure
sein.

Die Erfindung betrifft auch Verfahren zur Herstellung von Verbindungen der Formel I, die dadurch gekennzeichnet sind , daß man
a) Verbindungen der allgemeinen Formel V

   (V) A-(OH)ₙ

   mit A und n = wie in Formel I
   mit Diisocyanat, vorzugsweise Hexamethylendiisocyanat, umsetzt und die daraus entstehende Verbindung mit Polyethyleniminen mit den allgemeinen Formeln III und IV umsetzt, oder
b) Verbindungen der allgemeinen Formel VI

   (VI) A-(NH₂)ₙ

   (mit A und n = wie in Formel I definiert)
   bei der Polymerisation von Ethylenimin dem Reaktionsansatz von Beginn der Polyreaktion an oder erst später im Verlauf der Polyreaktion zufügt, oder
c) Verbindungen der allgemeinen Formel VII

   (VII) A-(OS(O)₂R⁴)ₙ

   mit A wie. in Formel I und R⁴ = aliphatischer oder aromatischer Rest, bevorzugt p-Toluyl, Fluorid, Trifluormethyl oder Methyl,
   als Makroinitiator für die Polymerisation von Ethylenimin einsetzt.
   Verbindungen der Formel VI sind in verschiedenen Molekulargewichten kommerziell beispielsweise von Shearwater erhältlich.
   Verbindungen der allgemeinen Formel VII werden durch die Umsetzung von Verbindungen der allgemeinen Formel V mit Verbindungen der allgemeinen Formel VIII

   (VIII) Cl-S(O)₂ R⁴ (R⁴ wie oben definiert)

   erhalten.
   Die Erfindung betrifft weiterhin Verfahren zur Herstellung von Verbindungen der Formel II, die dadurch gekennzeichnet sind, daß man
d) Verbindungen der allgemeinen Formel IX

   (IX) D-OH

   (mit D wie in Formel II definiert)
   zunächst mit Diisocyanat, vorzugsweise Hexamethylendiisocyanat, und anschließend die erhaltene Verbindung mit linearem oder verzweigtem Polyethylenimin umsetzt. Gegebenfalls zum Schutz von OH-Gruppen eingeführte Schutzgruppen können in an sich bekannter Weise abgespalten werden (s. z.B. Büllesbach, Kontakte (Merck) 1/1980, S. 23 ff.)

Das unter a) beschriebene Verfahren führt man vorzugsweise so durch, daß man je endständige Hydroxygruppe des Polymerblocks A einen 4 bis 20fachen Überschuß an Diisocyanat, vorzugsweise Hexamethylendiisocyanat, einsetzt. Die Reaktion wird in Chloroform bei Temperaturen von Raumtemperatur bis Siedetemperatur des Lösungsmittels, vorzugsweise aber bei Siedetemperatur des Lösungsmittels durchgeführt. Die Reaktionsdauer wird zwischen 2 und 24 Stunden gewählt, vorzugsweise jedoch 4 Stunden. Die Polymerkonzentration im Reaktionsansatzes liegt zwischen 10 g/l und 500 g/l, vorzugsweise 100 g/l. Das Produkt wird durch Entfernen des Lösungsmittels bei vermindertem Druck und Entfernen des überschüssigen Diisocyanates durch mehrfache Extraktion mit Petrolether (Siedebereich: 40-60°C) isoliert. Dieses Zwischenprodukt wird je endständiger Hydroxygruppe der Ausgangsverbindung mit einem 3 bis 10fachen Überschuß an PEI-Makromoleküle umgesetzt. Die Reaktion wird in Chloroform bei Temperaturen von Raumtemperatur bis Siedetemperatur des Lösungsmittels, vorzugsweise aber bei Siedetemperatur des Lösungsmittels durchgeführt. Die Reaktionsdauer wird zwischen 6 und 72 Stunden gewählt, vorzugsweise jedoch 12 Stunden. Die Polymerkonzentrationen, sowohl die des PEI als auch die des mit Hexamethylendiisocyanat aktivierten nichtionische hydrophile Polymer im Reaktionsansatzes liegen zwischen 10 g/l und 500 g/l, vorzugsweise zwischen 30 - 200 g/l. Das Produkt wird durch Ausfällen des Polymers in einem 10 - 30fachen Volumenüberschuß an Diethylether isoliert. Durch mehrmaliges Umfällen mit den Lösungsmittel Ethanol und Diethylether läßt sich das Blockcopolymer von überschüssigem PEI reinigen.

Das unter b) beschriebene Verfahren führt man durch, indem sowohl das Ethylenimin als auch das aminoterminierte hydrophile nichtionische Polymer in Wasser in einer Konzentration von jeweils 10 g/l bis 500 g/l vorgelegt werden. Das Molverhältnis der beiden Komponenten liegt zwischen 1 : 10 bis 1: 10.000. Dann wird die Ethylenimin-Polymerisation durch Zugabe eines geeigneten Katalysator, beispielsweise Salzsäure, initiiert und der Ansatz auf eine Temperatur von 40 - 100°C gebracht. Das Copolymer wird durch eine Kettenabbruchreaktion erzeugt. Das Blockcopolymer wird durch mehrmaliges Umfällen mit Hilfe geeigneter Lösungsmittel, beispielsweise Ethanol und Diethylether und/oder durch Druckfiltration von möglicherweise als Nebenprodukt entstandenes Homopolymer PEI gereinigt. Eine Variation dieses Herstellungsverfahrens besteht darin, daß man das aminoterminierte hydrophile nichtionische Polymer erst nach einer gewissen Reaktionsdauer von 30 Minuten bis zu 72 Stunden zu dem heißen Polymerisationsansatz zugibt.

Das unter c) beschriebene Verfahren führt man durch, indem man die endständige(n) Hydroxygruppe(n) des Polymerblocks A mit einem Sulfonsäurechlorid der allgemeinen Formel VIII, insbesondere jedoch mit Toluolsulfonsäurechlorid (Tosylchlorid) umsetzt. Diese Reaktion wird in wäßrigen und/oder polaren organischen Lösungsmittel, vorzugsweise in einem Wasser/Tetrahydrofuran-Gemisch, bei Temperaturen von -10°C bis Siedetemperatur des Lösungsmittel, bevorzugt bei Temperaturen von 0°C bis 25°C, und (falls nötig) in Gegenwart von Katalysatoren, wie beispielsweise Triethylamin oder Natriumhydroxid durchgeführt. Durch Entfernen des Lösungsmittels bei vermindertem Druck wird das Produkt isoliert. Dieses Polymer dient im folgenden als Makroinitiator bei der Ethylenimin-Polymerisation. Dazu wird das Produkt mit der allgemeinen Formel VII mit Ethylenimin in wäßrigen oder polaren organischen Lösungsmittel bei Temperaturen von 0°C bis Siedetemperatur des Lösungsmittel umgesetzt. Das Molverhältnis der beiden Komponenten liegt zwischen 1 : 10 bis 1: 10.000. Bei dieser Reaktion entsteht kein Nebenprodukt. Das Endprodukt läßt sich durch Fällen des Polymers in einem geeignetem Lösungsmittel, wie beispielsweise Diethylether isolieren. Das unter d) beschriebene Verfahren führt man vorzugsweise so durch, daß man eine Verbindung mit der allgemeinen Formel IX mit einem geringen Überschuß, beispielsweise einen 2 bis 10fachen Überschuß an Diisocyanat, vorzugsweise Hexamethylendiisocyanat, umsetzt. Die Reaktion wird in Chloroform bei Temperaturen von 20°C bis Siedetemperatur des Lösungsmittels, vorzugsweise aber bei Siedetemperatur des Lösungsmittels durchgeführt. Die Reaktionsdauer wird zwischen 2 und 24 Stunden gewählt, vorzugsweise jedoch 10 bis 14 Stunden. Die Polymerkonzentration im Reaktionsansatzes liegt zwischen 10 g/l und 500 g/l, vorzugsweise 30 bis 150 g/l. Das Produkt wird durch Entfernen des Lösungsmittels bei vermindertem Druck und Entfernen des überschüssigen Diisocyanates durch mehrfache Extraktion mit Petrolether (Siedebereich: 40-60°C) isoliert. Dieses Zwischenprodukt wird mit PEI-Makromolekül in einem Molverhältnis von 1 : 1 bis 100 : 1 umgesetzt. Die Reaktion wird in Chloroform und falls nötig mit einem Dimethylformamid-Zusatz bei Temperaturen von Raumtemperatur bis Siedetemperatur des Lösungsmittels, vorzugsweise aber bei 60 -70°C durchgeführt. Die Reaktionsdauer wird zwischen 6 und 72 Stunden gewählt, vorzugsweise jedoch 12 Stunden. Die Polymerkonzentrationen, sowohl die des PEI als auch die des mit Hexamethylendiisocyanat aktivierten nichtionischen hydrophilen Polymers im Reaktionsansatzes liegen zwischen 10 g/l und 500 g/l, vorzugsweise zwischen 30 - 200 g/l. Das Produkt wird durch Ausfällen des Polymers in einem 10 - 30fachen Volumenüberschuß an Diethylether isoliert.

Im Vergleich zu PEI weisen die neuen Verbindungen folgende Eigenschaften auf:

Die Blockcopolymere weisen in Cytotoxizitätstests eine geringere Toxizität auf als PEI-Homopolymere und bleiben länger in Blutzirkulation (siehe Abschnitt "Biologische Beispiele").

Die Blockcopolymere sind je nach Struktur mehr oder weniger oberflächenaktive Substanzen, die als Tenside Verwendung finden können.

Darüber hinaus können die Blockcopolymere auch
- in Klebstoff- und Lacksystemen als Additiv
- als Fixiermittel zur Verbesserung der Papierfestigkeit
- als Primer für Polymerverbundsysteme wie z. B. mehrlagige Verpackungsfolien
- zur Kunstoffmodifizierung (Verbesserung der Anfärbbarkeit, Lackierbarkeit, Barrierewirkung)
- zur Fixierung von Reaktivfarbstoffen auf Baumwolle
- als Flockungs- und Dispergiermittel bei feinverteilten Schwebstoffen in industriellen Abwässern
- zur Bindung von Schwermetallsalzen
- zur Dispergierung organischer und anorganischer Pigmente
- als Zusatz in keramischen und zementösen Bestanteilen
- für verschiedenste Funktionen in der Haut- und Haarkosmetik sowie im Dentalbereich
- zur Fixierung von medizinischen Wirkstoffen oder biologisch aktiven Verbindungen auf Oberflächen
- zur Filtration von Endotoxinen und Pathogenen aus Blutplasma
- zur Penetration durch Schleimhäute

verwendet werden.

Zudem bilden die Blockcopolymere in wäßrigen Systemen Komplexe mit Polynukleinsäuren, wie DNA und RNA einschließlich Ribozymen. Aufgrund dieser Eigenschaft sind sie geeignet als Vehikel oder Vektoren für den Gentransfer (Penetration durch Zellmembranen und Translokation in den Zellkern). Sie können daher in Transfektionsexperimenten, in der Gentherapie und Diagnostik Anwendung finden (siehe Abschnitt "Biologische Beispiele").

Die Erfindung betrifft daher insbesondere auch die Verwendung von Verbindungen der Formel I oder II, die wie oben definiert sind und in denen darüber hinaus Y für -C(O)NH(CH2)ₛNHC(O)O- mit s = 1 bis 20, vorzugsweise 2 bis 10, insbesondere 4 bis 6 stehen kann, zur Komplexierung von Polynukleinsäuren, wie DNA, RNA und Ribozymen, in wäßrigen Systemen, sowie Zusammensetzungen, die mindestens eine solche Nukleinsäure und eine Verbindung der Formel I oder II umfassen.

Die Erfindung betrifft auch die Verwendung der im vorstehenden Absatz definierten Verbindungen der Formel I oder II als Tenside.

Die folgenden Beispiele dienen zur Erläuterung der Erfindung, ohne daß diese darauf beschränkt wäre.

### Chemische Beispiele

### Beispiel 1:

### Darstellung eines PEI(PEG)ₙ-Blockcopolymers

### Aktivierung von mPEG-550

In einem 100 ml Rundkolben mit Magnetrührstab, Rückflußkühler und aufgesetztem Trockenrohr werden 10 ml Chloroform vorgelegt und mit 7 ml Hexamethylendiisocyanat (HMDI) (43,64 mmol, 8 eq.) versetzt. 3 g Polyethylenoxidmonomethylether (mPEG, Mₙ = 550 g/mol) ( 5,45 mmol, 1 eq.) werden in 40 ml Chloroform gelöst. Diese Lösung wird nun langsam unter Rühren zu der HMDI-Lösung getropft. Der Ansatz wird unter Rückfluß für 12 Stunden erhitzt. Anschließend wird das Lösungsmittel unter vermindertem Druck entfernt und das überschüssige HMDI mit Petrolether (40-60) (5 mal 50 ml) extrahiert. In nahezu quantitativer Ausbeute (3,8 g, 97%) wird das Produkt als farbloses dünnflüssiges Öl erhalten.

### Darstellung eines PEI-graft-PEG Blockcopolymers

In einem 100 ml Rundkolben mit Magnetrührstab, Rückflußkühler und aufgesetztem Trockenrohr werden 1,74 g bPEI (M_{w} = 25 kDa, Mₙ = 10 kDa, 0,1736 mmol, 1 eq.) eingewogen und mit 40 ml Dimethylformamid (DMF) in Lösung gebracht. 2,5 g des mit HMDI aktivierten mPEGs (Mₙ =720 Da, 3,47 mmol) werden in 10 ml Chloroform gelöst und diese Lösung wird unter Rühren langsam zur PEI-Lösung getropft. Der Ansatz wird für 12 Stunden auf 60 - 70 °C erhitzt. Anschließend wird der Ansatz in 500 ml Diethylether getropft. Nach zwei Stunden hat sich ein zähflüssiges gelbliches Öl abgesetzt. Der trübe Überstand wird verworfen und das Öl in 30 ml Ethanol gelöst. Die Lösung wird wiederum in 500 ml Diethylether getropft und das erneut ausgefallene Öl durch Dekantieren isoliert. Das Produkt wird zum Filtrieren in Ethanol gelöst und im Vakuumtrockenofen bei 50°C von Lösungsmittel befreit. Es werden 2,8 g eines gelblichen zähflüssigen bis harzartigen Öls erhalten (Ausbeute: 45%).

Die Charakterisierung der Polymere erfolgte mittels ¹H-, ¹³C-NMR-Spektrokopie und Gelpermeationschromatographie. Die nachfolgenden Daten wurden für das Beispiel Nr. 1 erhoben. Sie gelten exemplarisch für die anderen Beispiele, für die ähnliche Daten erhoben worden sind.

¹H-NMR (500 MHz, CDCl₃): δ/ppm = 1,17 (Isocyanat-CH₂), 1,26 (isocyanat-CH₂), 2,30 - 2,72 (Ethylenimin-CH₂), 2,96 (Isocyanat-CH₂), 3,15 (Isocyanat-CH₂), 3,49 Ethylenglykol-CH₂).

¹³C-NMR (125 MHz, CDCl₃): δ/ppm = 14,3 (isocyanat-CH₂), 26,2 (lsocyanat-CH₂), 29,6 (Isocyanat-CH₂), 36,2 (lsocyanat-CH₂), 37,5 (Ethylenimin-CH₂), 39,1 (Ethylenimin-CH₂), 41,1 (lsocyanat-CH₂), 47,2 (Ethylenimin-CH₂), 48,9 (Ethylenimin-CH₂), 52,8 (Ethylenimin-CH₂), 54,1 (Ethylenimin-CH₂), 58,7 (Isocyanat-CH₂), 69,3 und 70,2 und 71,6 (Ethylenglykol-CH₂), 156,2 (-NHC(O)O-), 161,7 (-NHC(O)NH-).

GPC (Aminoethylmethacrylatgel, 1% Ameisensäure, 0,5 ml/min, 25°C, geeicht gegen Pullulan-Standards): Mₙ = 8800, M_{w} = 1640000, Mₚ = 85000, PD = 19,6, monomodal. Vergleich mit Blend aus PEI (Aldrich, 25kDa) und mPEG (Aldrich,550 Da): Mₙ = 69000, M_{w} = 1480000, Mₚ = 99000 und 1100, PD = 2,1, bimodal.

Untersuchungen zur Grenzflächenaktivität des Polymers von Beispiel Nr. 1 wurden mit einen Tensiometer nach der Methode von Lecomte du Nouy (Ringmethode) bei 22°C durchgeführt. Es wurde die Grenzflächenspannung der Lösung gegen Luft gemessen. Die Eichung des Gerätes wurde mit Reinstwasser vorgenommen, das auch als Lösungsmittel für die Polymer-Probe eingesetzt wurde.
Meßdaten: σₘᵢₙ = 51 mN/m, CMC = 15 mg/ml.

In gleicher Weise können hergestellt werden: (alle Ausgangsverbindungen sind von Aldrich erhältlich)

| Nr. | Ausgangsverbindungen / Homopolymere | | | |
|---|---|---|---|---|
| | Polyethylenimin | Hydrophiles, nicht ionisches Polymer | Molverhältnis PEI:PEG | Struktur des Blockcopolymers |
| 2 | IPEI Mₙ ca. 423 | mPEG Mₙ ca. 550 | 1 : 1 | AB-Diblock |
| 3 | | | 1 : 2 | ABA-Triblock |
| 4 | bPEI M_{w} ca. 800 | mPEG Mₙ ca. 550 | 1 : 1 | AB-Diblock |
| 5 | | | 1 : 2 | ABA-Triblock |
| 6 | | | 1 : 4 | sternförmig |
| 7 | bPEI M_{w} ca. 800 | mPEG Mₙ ca. 5.000 | 1 : 1 | AB-Diblock |
| 8 | | | 1 : 2 | ABA-Triblock |
| 9 | | | 1 : 4 | sternförmig |
| 10 | bPEI M_{w} ca. 2.000 | mPEG Mₙ ca. 550 | 1 : 1 | AB-Diblock |
| 11 | | | 1 : 2 | ABA-Triblock |
| 12 | | | 1 : 4 | sternförmig |
| 13 | bPEI M_{w} ca. 2.000 | mPEG Mₙ ca. 5.000 | 1 : 1 | AB-Diblock |
| 14 | | | 1 : 2 | ABA-Triblock |
| 15 | bPEI M_{w} ca. 25.000 | mPEG Mₙ ca. 550 | 1 : 1 | AB-Diblock |
| 16 | | | 1:2 | ABA-Triblock |
| 17 | | | 1 : 4 | sternförmig |
| 18 | | | 1 : 20 | sternförmig |
| 19 | bPEI M_{w} ca. 25.000 | mPEG Mₙ ca. 2.000 | 1 : 1 | AB-Diblock |
| 20 | | | 1 : 2 | ABA-Triblock |
| 21 | | | 1 : 4 | sternförmig |
| 22 | | | 1 : 10 | sternförmig |
| 23 | bPEI M_{w} ca. 25.000 | mPEG Mₙ ca. 5.000 | 1 : 1 | AB-Diblock |
| 24 | | | 1 : 2 | ABA-Triblock |
| 25 | | | 1 : 4 | sternförmig |
| 26 | | | 1 : 10 | sternförmig |

### Beispiel 27: Darstellung eines PEG(PEI)ₙ-Blockcopolymers

### Aktivierung des verzweigten PEGs

In einem 100 ml Rundkolben mit Magnetrührstab, Rückflußkühler und aufgesetztem Trockenrohr werden 3,79 g HMDI (22,54 mmol, 80 eq.) in 10 ml Chloroform gelöst. Eine Lösung von 2 g eines achtarmig verzweigten PEG (bPEG, MW = 10 kDa, 0,2 mmol, 1 eq.) in 20 ml Chloroform wird unter Rühren langsam zu der HMDI-Lösung getropft. Der Ansatz wird für 4 Stunden zum Sieden erhitzt und bei Raumtemperatur 8 weitere Stunden nachgerührt. Unter vermindertem Druck wird das Lösungsmittel entfernt und das überschüssige HMDI mit Petrolether (40-60) extrahiert (3 mal 50 ml). Mit 58%iger Ausbeute (1,38 g) wird ein rötliches Öl erhalten.

### Darstellung eines PEG-graft-PEI Blockcopolymeren

In einem 100 ml Rundkolben mit Magnetrührstab, Rückflußkühler und aufgesetztem Trockenrohr werden 2,20 g eines verzweigten PEls (bPEI, M_{w} = 800 Da, Mₙ = 600 Da, 3,66 mmol, 25 eq.) in 20 ml Chloroform gelöst. Eine Lösung von 1,21 g des mit HMDI aktivierten bPEGs (Mₙ = 8,5 kDa, 0,14 mmol, 1 eq.) in 30 ml Chloroform wird bei Raumtemperatur zur PEI-Lösung unter Rühren langsam zugetropft. Der Ansatz wird für 12 Stunden zum Sieden erhitzt. Die Lösung wird dann in 500 ml Diethylether langsam unter Rühren eingetropft. Nach 12 Stunden hat sich ein zähflüssiges gelbliches Öl abgesetzt. Der trübe Überstand wird verworfen und das Öl in 50 ml Ethanol gelöst. Die Lösung wird wiederum in 500 ml Diethylether getropft und das erneut ausgefallene Öl durch Dekantieren isoliert. Das Produkt wird zum Filtrieren in Ethanol gelöst und im Vakuumtrockenofen bei 50°C von Lösungsmittel befreit. Es werden 1,13 g eines gelblichen zähflüssigen bis harzartigen Öls erhalten (Ausbeute: 59%).

Die Charakterisierung der Polymere erfolgte mittels ¹H-, ¹³C-NMR-Spektrokopie und Gelpermeationschromatographie. Die nachfolgenden Daten wurden für das Beispiel Nr. 27 erhoben. Sie gelten exemplarisch für die anderen Beispiele, für die ähnliche Daten erhoben worden sind.

¹H-NMR (500 MHz, CDCl₃): δ/ppm = 1,22 (Isocyanat-CH₂), 1,36 (Isocyanat-CH₂), 2,40-2,70 (Ethylenimin-CH₂), 3,03 (Isocyanat-CH₂), 3,19 (Isocyanat-CH₂), 3,55 Ethylenglykol-CH₂).

¹³C-NMR (125 MHz, CDCl₃): δ/ppm = 25,9 (Isocyanat-CH₂), 29,4 (Isocyanat-CH₂), 39,2 (Ethylenimin-CH₂), 41,2 (Isocyanat-CH₂), 47,0 (Ethylenimin-CH₂), 48,9 (Ethylenimin-CH₂), 52,0 (Ethylenimin-CH₂), 54,2 (Ethylenimin-CH₂), 61,1 (Isocyanat-CH₂), 69,2 und 71,1 und 72,3 (Ethylenglykol-CH₂), 156,0 (-NHC(O)O-), 162,1 (-NHC(O)NH-).

GPC (Aminoethylmethacrylatgel, 1 % Ameisensäure, 0,5 ml/min, 25°C, geeicht gegen Pullulan-Standards): Mₙ = 22000, M_{w} = 43000, Mₚ = 31000, PD = 1,9, monomodal. Vergleich mit Blend aus 8arm PEG (Shearwater, 10 kDa) und PEI (Aldrich,800 Da): Mₙ = 3100, M_{w} = 15000, Mₚ = 12000, PD = 4,91, monomodal.

Untersuchungen zur Grenzflächenaktivität des Polymers von Beispiel Nr. 27 wurden mit einen Tensiometer nach der Methode von Lecomte du Nouy (Ringmethode) bei 22°C durchgeführt. Es wurde die Grenzflächenspannung der Lösung gegen Luft gemessen. Die Eichung des Gerätes wurde mit Reinstwasser vorgenommen, das auch als Lösungsmittel für die Polymer-Probe eingesetzt wurde.
Meßdaten: σₘᵢₙ = 56 mN/m, CMC = 12 mg/ml.

In gleicher Weise können hergestellt werden:

| Nr. | Ausgangsverbindungen / Homopolymere | | |
|---|---|---|---|
| | Hydrophiles, nicht ionisches Polymer | Polyethylenimin | Struktur des Blockcopolymers |
| 28 | MPEG Mₙ ca. 5.000 (Aldrich) | IPEI Mₙ ca. 423 (Aldrich) | AB |
| 29 | | bPEI Mₙ ca. 800 (Aldrich) | AB |
| 30 | | bPEI Mₙ ca. 2.000 (Aldrich) | AB |
| 31 | LPEG Mₙ ca. 5.000 (Aldrich) | IPEI Mₙ ca. 423 (Aldrich) | ABA |
| 32 | | bPEI Mₙ ca. 800 (Aldrich) | ABA |
| 33 | | bPEI Mₙ ca. 2.000 (Aldrich) | ABA |
| 34 | 4armPEG MW ca. 15.000 (Shearwater) | IPEI Mₙ ca. 423 (Aldrich) | AB₄ |
| 35 | | bPEI Mₙ ca. 800 (Aldrich) | AB₄ |
| 36 | | bPEI Mₙ ca. 2.000 (Aldrich) | AB₄ |
| 37 | 8armPEG MW ca. 10.000 (Shearwater) | IPEI Mₙ ca. 423 (Aldrich) | AB₈ |
| 38 | | bPEI Mₙ ca. 800 (Aldrich) | AB₈ |
| 39 | | bPEI Mₙ ca. 2.000 (Aldrich) | AB₈ |
| 40 | Star-PEG 429 MW ca. 250.000 (Shearwater) | IPEI Mₙ ca. 423 (Aldrich) | AB₁₃ |
| 41 | | bPEI Mₙ ca. 800 (Aldrich) | AB₁₃ |
| 42 | | bPEI Mₙ ca. 2.000 (Aldrich) | AB₁₃ |
| 43 | α-Cyclodextrin (Aldrich) | IPEI Mₙ ca. 423 (Aldrich) | AB₁₈ |
| 44 | | bPEI Mₙ ca. 800 (Aldrich) | AB₁₈ |
| 45 | | bPEI Mₙ ca. 2.000 (Aldrich) | AB₁₈ |
| 46 | β-Cyclodextrin (Aldrich) | IPEI Mₙ ca. 423 (Aldrich) | AB₂₁ |
| 47 | | bPEI Mₙ ca. 800 (Aldrich) | AB₂₁ |
| 48 | | bPEI Mₙ ca. 2.000 (Aldrich) | AB₂₁ |
| 49 | γ-Cyclodextrin (Aldrich) | IPEI Mₙ ca. 423 (Aldrich) | AB₂₄ |
| 50 | | bPEI Mₙ ca. 800 (Aldrich) | AB₂₄ |
| 51 | | bPEI Mₙ ca. 2.000 (Aldrich) | AB₂₄ |
| 52 | PVA 80% hydrolysiert | IPEI Mₙ ca. 423 (Aldrich) | ABₙ |
| | M_{w} = 9.000 - 10.000 | | |
| 53 | | bPEI Mₙ ca. 800 (Aldrich) | ABₙ |
| 54 | | bPEI Mₙ ca. 2.000 (Aldrich) | ABₙ |

### Beispiel 55:

### Darstellung eines PEG-PEI-Copolymers (Makroreglerroute)

In einem 50 ml Rundkolben mit Magnetrührstab und Rückflußkühler werden 1 g (0,2 mmol) eines monomethylierten PEG (MW 5000 g/mol), das an dem anderen Kettenende eine Aminogruppe trägt, eingewogen und in 20 ml destilliertem Wasser gelöst. Zu dieser Polymerlösung werden 2 ml (39 mmol) Ethylenimin zugesetzt. Die Polymerisation wird mit 200 µl (2 mmol) Dimethylsulfat als Initiator gestartet und der Ansatz für 8 Tage auf 60°C erhitzt. Unter vermindertem Druck wird dann das Lösungsmittel entfernt, um die zurückbleibende Masse in 20 ml Ethanol wieder aufzulösen. Die Lösung wird in 250 ml Diethylether eingetropft, wobei das Polymer ausfällt. Durch Filtration wird das Polymer isoliert und für 3 Wochen bei 50 °C im Vakuumtrockenschrank von Lösungsmittelresten befreit. Es werden 1,9 g eines schwach gelblichen, harzartigen Polymers erhalten (Ausbeute: 73%).

In gleicher Weise können hergestellt werden: (alle aminomodifizierten PEG sind von RAPP Polymere, Tübingen erhältlich)

| Nr. | Ausgangsverbindungen | | | |
|---|---|---|---|---|
| | Polyethylenglykol | Polyethylenimin | Molverhältnis EG : EI | Struktur des Blockcopolymers |
| 56 | CH₃O-PEG-NH₂ Mₙ ca. 2000 | Ethylenimin | 1 : 1 | AB-Diblock |
| 57 | | | 1 : 2 | AB-Diblock |
| 58 | | | 1 : 10 | AB-Diblock |
| 59 | CH₃O-PEG-NH₂ Mₙ ca. 5000 | Ethylenimin | 1 : 1 | AB-Diblock |
| 60 | | | 1 : 10 | AB-Diblock |
| 61 | CH₃O-PEG-NH₂ Mₙ 10.000 | Ethylenimin | 1 : 1 | AB-Diblock |
| 62 | | | 1 : 2 | AB-Diblock |
| 63 | | | 1 : 10 | AB-Diblock |
| 64 | CH₃O-PEG-NH₂ Mₙ 20.000 | Ethylenimin | 1 : 1 | AB-Diblock |
| 65 | | | 1 : 2 | AB-Diblock |
| 66 | | | 1 : 10 | AB-Diblock |

Die Charakterisierung der Polymere erfolgte mittels ¹H-, ¹³C-NMR-Spektrokopie und Gelpermeationschromatographie. Die nachfolgenden Daten wurden für das Beispiel Nr. 56 erhoben. Sie gelten exemplarisch für die anderen Beispiele, für die sehr ähnliche Daten erhoben werden.

¹H-NMR (500 MHz, D₂O): δ/ppm = 2,60 - 3,00 (Ethylenimin-CH₂), 3,78 (Ethylenglykol-CH₂).

¹³C-NMR (125 MHz, D₂O): δ/ppm = 38,2 (Ethylenimin-CH₂), 39,9 (Ethylenimin-CH₂), 46,2 (Ethylenimin-CH₂), 47,9 (Ethylenimin-CH₂), 51,7 (Ethylenimin-CH₂), 53,4 (Ethylenimin-CH₂), 54,8 (Ethylenimin-CH₂), 70,2 (Ethylenglykol-CH₂).

GPC (Aminoethylmethacrylatgel, 1% Ameisensäure, 0,5 ml/min, 25°C, geeicht gegen Pullulan-Standards): Mₙ = 21000, M_{w} = 40000, Mₚ = 16000, PD = 1,9, monomodal. Vergleich mit CH₃O-PEG-NH₂ (RAPP Polymere, 5000 Da): Mₙ = 9100, M_{w} = 14000, Mₚ = 16000, PD = 1,6, monomodal.

### Beispiel 67:

### Darstellung eines PEG-PEI-Copolymers (Makroinitiatorroute)

### Darstellung des Makroinitiators

In einem 50 ml Rundkolben mit Magnetrührstab und Rückflußkühler werden 2 g (0,4 mmol, 1 eq.) eines Monomethylether-Polyethylenglykols (Aldrich, MW 5000) eingewogen und in 25 ml destilliertem Chloroform gelöst. 0,31 g Tosylchlorid (1,6 mmol, 4 eq.) werden der Polymerlösung unter Rühren zugesetzt. Abschließend werden 0,22 ml Triethylamin (0,16 g, 1,6 mmol, 4 eq.) als Katalysator dem Ansatz zugegeben. Für 18 h wird der Ansatz unter Rückfluß erhitzt. Zur Isolierung und Reinigung des Polymers wird die Lösung in 500 ml Diethylether eingegossen. Das ausgefällte Polymer wird abfiltriert, mit reichlich Diethylether nachgewaschen und im Vakuum getrocknet. Es werden 1,90 g einer weißen, flockigen Substanz erhalten (91 % Ausbeute).

### Darstellung des PEG-PEI-Blockcopolymers

In einem 25 ml Rundkolben mit Magnetrührstab und Rückflußkühler werden 0,5 g des Makroinitiators (0,096 mmol, 1 eq.) eingewogen und in 10 ml destilliertem Wasser gelöst. Unter Rühren wird 1 ml Ethylenimin (0,832 g, 19,32 mmol, 200 eq.) zugetropft und der Ansatz für 24 h auf 60°C erhitzt. Die flüchtigen Bestandteile werden unter vermindertem Druck entfernt. Zurück bleibt eine weiße, harzartige Substanz, die mit 10 ml Wasser wieder in Lösung gebracht und mit 200 ml Tetrahydrofuran gefällt wird. Das Polymer wird durch Dekantieren isoliert und im Vakuum getrocknet. Es werden 0,95 g einer gelblichen, harzartigen Substanz erhalten (71% Ausbeute).

In gleicher Weise können hergestellt werden: (alle Monomethyl-PEG sind von Aldrich erhältlich)

| Nr. | Ausgangsverbindungen | | | |
|---|---|---|---|---|
| | Polyethylenglykol | Polyethylenimin | Molverhältnis PEG : EI | Struktur des Blockcopolymers |
| 68 | CH₃O-PEG-Ts Mₙ ca. 550 | Ethylenimin | 1 : 10 | AB-Diblock |
| 69 | | | 1 : 50 | AB-Diblock |
| 70 | | | 1 : 200 | AB-Diblock |
| 71 | CH₃O-PEG-Ts Mₙ ca. 750 | Ethylenimin | 1 : 10 | AB-Diblock |
| 72 | | | 1 : 50 | AB-Diblock |
| 73 | | | 1 : 200 | AB-Diblock |
| 74 | CH₃O-PEG-Ts Mₙ ca. 2.000 | Ethylenimin | 1 : 10 | AB-Diblock |
| 75 | | | 1 : 50 | AB-Diblock |
| 76 | | | 1 : 200 | AB-Diblock |
| 77 | CH₃O-PEG-Ts Mₙ ca. 5.000 | Ethylenimin | 1 : 10 | AB-Diblock |
| 78 | | | 1 : 50 | AB-Diblock |

Die Charakterisierung der Polymere erfolgte mittels ¹H-, ¹³C-NMR-Spektrokopie und Gelpermeationschromatographie. Die nachfolgenden Daten wurden für das Beispiel Nr. 67 erhoben. Sie gelten exemplarisch für die anderen Beispiele, für die sehr ähnliche Daten erhoben werden.

¹H-NMR (500 MHz, D₂O): δ/ppm = 2,80 - 3,20 (Ethylenimin-CH₂), 3,80 (Ethylenglykol-CH₂).

¹³C-NMR (125 MHz, D₂O): δ/ppm = 37,9 (Ethylenimin-CH₂), 39,4 (Ethylenimin-CH₂), 46,1 (Ethylenimin-CH₂), 47,2 (Ethylenimin-CH₂), 51,3 - 52,7 (Ethylenimin-CH₂), 70,2 (Ethylenglykol-CH₂).

GPC (Aminoethylmethacrylatgel, 1% Ameisensäure, 0,5 ml/min, 25°C, geeicht gegen Pullulan-Standards): Mₙ = 35000, M_{w} = 90000, Mₚ = 52000, PD = 2,6, monomodal. Vergleich mit CH₃O-PEG-Ts 5000 Da): Mₙ = 4800, M_{w} = 7600, Mₚ = 8600, PD = 1,6, monomodal.

### Abkürzungen

- bPEG: Verzweigtes Polyethylenglykol (branched)
- bPEI: Verzweigtes Polyethylenimin (branched)
- CMC: Kritische Mizellbildungskonzentration
- DMF: Dimethylformamid
- HMDI: Hexamethylendiisocyanat
- IPEG: Lineares Polyethylenglykol (linear)
- IPEI: Lineares Polyethylenimin (linear)
- Mₙ: Zahlenmittel des Molekulargewichts
- Mₚ: Peak-Molekulargewicht
- mPEG: Monomethoxypolyethylenglykol
- M_{w}: Gewichtsmittel des Molekulargewichts
- MW: Unbestimmtes Molekulargewichtsmittel
- PD: Polydispersität
- Ts: Tosyl-
- σₘᵢₙ: minimale Oberflächenspannung

### Biologische Beispiele

### I. Transfektionsexperimente

Die Transfektionseigenschaften der Polymere PEI(PEG)₂₀ (Beispiel 1) und PEG(PEI)₈ (Beispiel 27) wurden an der Zellinie 3T3 untersucht. 50000 Zellen/well wurden in 12 well plates ausgesät und für 24 Stunden inkubiert (DMEM + 2mM Glutamin + 10% FKS, 37°C, 10% CO₂). Dann wurde das Medium gewechselt. Pro well wurden jeweils 4 µg pGL3-Plasmid in 100 µl 150mM Kochsalzlösung mit der entsprechenden Menge Polymer in 100 µl 150mM Kochsalzlösung komplexiert und nach 10 Minuten zu den Zellen gegeben. Nach 4 Stunden wurde das Medium erneut gewechselt, nach 48 Stunden erfolgte die Auswertung. Die Luciferase-Expression wurde mit dem Luciferase Assay Kit von Promega auf einem Berthold Sirius Luminometer bestimmt. Die Proteinkonzentration wurde mit einem modifizierten BCA Assay quantifiziert. Die angegebenen Daten sind jeweils der Mittelwert von drei wells ± Standardabweichung für die entsprechenden Stickstoff / Phosphor Quotienten.

### Beispiel 1: [PEI(PEG)₂₀]

**Meßdaten:**

| | |
|---|---|
| N/P 5 : | 0,0057 ± 0,0036 ng / mg Protein |
| N/P 10: | 0,1786 ± 0,1522 ng / mg Protein |
| N/P 20 : | 0,6952 ± 0,5498 ng / mg Protein |
| N/P 50 : | 5,1963 ± 2,6863 ng / mg Protein |

| | |
|---|---|
| (nur Plasmid: 0,0000 ± 0,00004 ng / mg Protein) | |

### Beispiel 27: [PEG(PEI)₈]

**Meßdaten:**

| | |
|---|---|
| N/P 5 : | 0,0024 ± 0,0012 ng / mg Protein |
| N/P 10 : | 0,0045 ± 0,0046 ng / mg Protein |
| N/P 20 : | 0.0109 ± 0,0078 ng / mg Protein |
| N/P 50 : | 0,0765 ± 0,0498 ng / mg Protein |

| | |
|---|---|
| (nur Plasmid: 0,0000 ± 0,00004 ng / mg Protein) | |

In beiden Fällen konnte Genexpression aufgrund erfolgter Transfektion detektiert werden. Dabei weist das PEI(PEG)₂₀ eine deutlich höhere Transfektionseffizienz als das PEG(PEI)₈ auf.

### II. In vitro-Zytotoxizitäts-Bestimmung mittels MTT-Assay:

Die Copolymere der Beispiele 1 und 27 wurden bezüglich ihrer Zytotoxizität im Zellkulturmodell mittels MTT-Assay nach der Methode von Mosmann (J. Immunol. Methods. 65: 55-63 (1983)) untersucht. 8000 L929 Maus-Fibroblasten/well wurden in 96-wells 24 h lang vorinkubiert und mit den Polymerlösungen der verschiedenen Konzentrationen 3, 12 und 24 h behandelt. Die Bestimmung der mitochondrialen Aktivität erfolgte durch die Umsetzung des MTT-Farbstoffs zum Formazan, welches spektrophotometrisch quantifiziert wurde. Die Polymere wurden als Lösungen in DMEM mit 10% FKS in fünf verschiedenen Konzentrationen eingesetzt. Falls erforderlich, wurde der pH-Wert auf 7,4 eingestellt und die Proben sterilfiltriert (0,2 µm). Die Blends wurden durch Mischen der beiden Einzelkomponenten (abzüglich der Spacermenge) hergestellt. Zur Auswertung wurde die zelluläre Viabilität [%] gegen die eingesetzten Polymerkonzentrationen aufgetragen sowie die IC50 bestimmt.

### Ergebnis:

- Die in vitro-Zytotoxizität der freien Polymere steigt mit steigender Polymerkonzentration und der Zunahme der Inkubationsdauer an.
- Copolymer des Beispiels 1: Die Toxizität der Mischung der Einzelkomponenten PEI 25 kDa und PEG 550 Da entspricht der Toxizität des freien PEI 25 kDa. Durch die kovalente Verknüpfung der beiden Komponenten verbessert sich die Verträglichkeit deutlich. Nach 24 h entspricht das Toxizitätsprofil zwar noch dem der Einzelkomponenten und damit dem freien PEI 25 kDa, bei kürzeren Inkubationsphasen dagegen sinkt die Zytotoxizität. Durch das PEG-Coating wird die positive Ladung des Polyethylenimins maskiert und damit die ladungsvermittelten Effekte auf die Zellmembranen reduziert.
- Copolymer des Beispiels 27: Die Mischung der beiden Einzelkomponenten PEI 700 Da und PEG 10 kDa zeigte bis zu 10 mg/ml keine Reduktion der Lebensfähigkeit der Zellen. Im gleichen Konzentrationsbereich zeigte das Copolymer eine erhöhte Einschränkung der zellulären Viabilität nach 3, 12 und 24 h, was mit dem Molekulargewichtsaufbau zu erklären ist.
- Im Vergleich weist Beispiel 27 eine geringere Zytotoxizität als Beispiel 1 auf.

### II. In vitro-Zytotoxizitäts-Bestimmung mittels LDH-Assay:

L929 Maus-Fibroblasten wurden in der gleichen Zelldichte wie beim MTT-Assay in 6-well Multischalen ausgesät, 48 h vorinkubiert und mit den Polymerlösungen (in PBS pH 7,4) 1, 2, 3 und 6 h inkubiert. Der extrazelluläre LDH-Anteil wurde quantifiziert mit einem Standard-Kit (Sigma, DG-1340-K) durch photometrische Bestimmung der Reduktion von NAD in Gegenwart von Lactat und LDH. Zur Bestimmung des 100%-Wertes wurden Zellen mit 0,1 % Triton-X 100 lysiert.

### Ergebnis:

Der LDH-Assay bestätigt die Ergebnisse des MTT-Tests. Die Korrelation der beiden Assays zeigt, daß zunächst die Membranschädigung und zeitlich verzögert die Reduktion der metabolischen Aktivität einsetzt. Mit steigender Inkubationsdauer und Polymerkonzentration verstärkt sich die membranschädigende Wirkung der Polymere.

### IV. DNA-Bindung der Copolymere bestimmt mittels Agarosegel-Elektrophorese:

Das Bindungsvermögen der Copolymere der Beispiele 1 und 27 wurde mit 1%igen Agarosegelen bei 80 V elektrophoretisch bestimmt. Die Lokalisierung der Plasmide (CMV-nlacZ) erfolgt mittels UV-Anregung bei 254 nm nach Ethidiumbromidfärbung.

### Ergebnis:

- Beide Polymere sind in der Lage elektrostatisch mit dem Plasmid zu interagieren.
- Übereinstimmend mit dem Blend ist das Polymer des Beispiels 1 in der Lage Plasmid vollständig zu binden ab einem Stickstoff-PEI/Phosphat-DNA-Verhältnis (N/P-Ratio) von 1,7. Die beim Blend beobachtete Ethidiumbromidexklusion (ab N/P 5,8), ein Zeichen für intensive DNA-Kondenstation, ist bis N/P 23,0 für das Copolymer unvollständig.
- Während für den Blend des Beispiels 27 eine vollständige Plasmidbindung erst ab N/P 4,1 und keine völlige Ethidiumexklusion zu beobachten ist, zeigte das Copolymer Plasmidbindung ab N/P 2,4 und Exklusion des Farbstoffes ab N/P 16,6.

### V. Erythrozyten-Aggregations-Assay

Erythrozyten wurden aus dem Citratblut von Wistar-Ratten nach der Methode von Parnham und Wetzig (Chem. Phys. Lipids, 1993, 64: 263-274) isoliert, in 24-Wells ausgesät und 2 h lang mit den Testlösungen bei 37°C inkubiert. Mikroskopisch wurde die Aggregation und Adhäsion der Erythrozyten unter dem Einfluß des Polymers untersucht. Als Kontrolle dienten unbehandelte Erythrozyten.

### Ergebnis:

- Freies Copolymer Beispiel 1 zeigte bei Konzentrationen von 0,27-18 µg/well im Vergleich zum Blend und zum PEI 25 kDa eine verringerte Aggregation und Adhäsion der roten Blutkörperchen an den Zellkulturschalen. Während bei niedrigen Konzentrationen (0.27-0,7 µg/well) keine signifikanten Unterschiede zu beobachten waren, ließ sich mit steigender Konzentration ein deutlicher Unterschied zwischen Copolymer und Blend bzw. PEI 25 kDa detektieren. Der Aggregationseffekt steigt bei steigendem N/P-Verhältnis.
- Copolymer Beispiel 27 zeigte das gegenteilige Verhalten. Die Aggregation des Blends bzw. des freien PEls ist schwächer ausgeprägt als die des Copolymers.
- Durch die Komplexierung mit Plasmid-DNA vermindert sich bei beiden Copolymeren die Aggregation der Erythrozyten signifikant im Vergleich zum freien Polymer.

### VI. Hämolyse-Assay

Erythrozyten wurden aus dem Citratblut von Wistar-Ratten nach der Methode von Parnham und Wetzig (Chem. Phys. Lipids, 1993, 64: 263-274) isoliert, mit den Polymerlösungen versetzt und 1 h lang bei 37°C inkubiert. Die Erythrozyten werden durch Zentrifugation pelletiert (10 min, 25°C, 700 g) und aus dem Überstand bei 540 nm das Hämolysat photometrisch vermessen.

### Ergebnis:

- Die Einzelkomponenten PEG 8-arm, PEG 500 Da und PEI 700 Da zeigen im Konzentrationsbereich 0,001-10 mg/ml keine signifikanten hämolytischen Effekte (alle 1-3%).
- Das Copolymer Beispiel 27 weist im gleichen Konzentrationsbereich ebenfalls keine ausgeprägten Effekte auf (<5%).
- Bei der Einzelkomponente PEI 25 kDa sowie dem Blend für Beispiel 1 steigt die hämolytische Aktivität bei 0,001-10 mg/ml an (22,13% bei 10 mg/ml).
- Das Copolymer Beispiel 1 zeigt bis zu 0,5 mg/ml eine ansteigende lytische Aktivität bis zu 13,30%, bei höheren Konzentration bis zu 10 mg/ml nimmt der hämolytische Effekt wieder ab (2,90% bei 10 mg/ml).

### VI Pharmakokinetik und Organverteilung von Polymer-DNA-Komplexen in der Maus

Pharmakokinetik und Organverteilung der Copolymere Beispiel 1 und 27 wurden in balb/c Mäusen bestimmt. Die Polymere wurden mit ¹²⁵I Bolton Hunter Reagenz (Pharmacia Biotech) radioaktiv markiert. Es wurden jeweils 0,4 bzw. 0,04 bzw. 0,008 mg PEI(-Anteil) pro kg Maus mit der entsprechenden Menge NF-κB decoy Oligodeoxynucleotid (ODN) im Stickstoff / Phosphor Verhältnis N/P 3,5 bzw. N/P 6 in einem Gesamtvolumen von 80 µl in 5% Glucoselösung komplexiert und nach 10 Minuten den narkotisierten Mäusen über die Schlüsselbeinvene injiziert. Nach 20 Sekunden, 1, 2, 5, 15, 30, 60, 90 und 120 Minuten wurden Blutproben über einen Katheter aus der Arteria Aorta communis entnommen. Über einen Blasenkatheter wurde der Urin über 120 Minutern gesammelt. Nach 120 Minuten wurden die Mäuse dekapitiert und die Organe Cortex, Niere, Leber, Herz, Lunge, Milz und Fettgewebe wurden entnommen. Die Polymermenge in den Proben wurde mittels Messung der Radioaktivität mit einem 1277 Gammamaster Automatic Gamma Counter (LKB Wallac) bestimmt.
Die Daten wurden mit dem Programm Kinetica 1.1 und einem 2-Kompartiment-Modell für i.v. Bolus-Injektion ausgewertet. Aus den Blutspiegelkurven wurden Verteilungsvolumen (V_{C}), die Konstante für Elimination (kₑₗ) und AUC berechnet. Bei drei auswertbaren Tieren sind Mittelwert ± Standardabweichung, bei zwei Tieren der Median angegeben, bei nur einem Tier ist der Wert in Klammern aufgeführt.

### Komplexherstellung und Dosierungen

| Polymer | N/P | Dosis [mg/kg] | V_{C} [ml] | kₑₗ [min⁻¹] | AUC [min µg ml⁻¹] |
|---|---|---|---|---|---|
| 25 kDa PEI | 3,5:1 | 0,4 | 23,39 | 0,106 | 4,89 |
| Beispiel 1 | 3,5:1 | 0,4 | (4,54) | (0,028) | (79,03) |
| Beispiel 27 | 3,5:1 | 0,4 | 5,84±0,4 | 0,104±0,017 | 16,86±1,64 |
| | | | | | |
| 25 kDa PEI | 6:1 | 0,4 | 5,39 | 0,099 | 19,22 |
| 25 kDa PEI | 6:1 | 0,04 | 1,37±0,2 | 0,14±0,026 | 6,22±1,18 |
| 25 kDa PEI | 6:1 | 0,008 | 9,57±1,78 | 0,063±0,009 | 0,34±0,1 |
| Beispiel 1 | 6:1 | 0,4 | 6,20 | 0,067 | 27,84 |
| Beispiel 1 | 6:1 | 0,04 | 3,37±0,32 | 0,072± 0,01 | 4,0±0,67 |
| Beispiel 1 | 6:1 | 0,008 | 5,1±0,55 | 0,054±0,004 | 0,80±0,10 |
| Beispiel 27 | 6:1 | 0,4 | 8,12 | 0,0593 | 21,72 |

### Ergebnis:

■ Beobachtungen bei niedrigerer Dosis deuten auf schwächere Toxizität des PEI(PEG)₂₀ gegenüber dem PEI 25 kDa hin.
■ Die Plasmaspiegel aller Polymere ließen sich mit einem 2-Kompartiment-Modell beschreiben.
■ Die Copolymere haben eine höhere AUC sowie ein kleineres Verteilungsvolumen als das 25 kDa PEI. PEI(PEG)₂₀ (Beispiel 1) hat einen größeren Effekt als PEG(PEI)₈ (Beispiel 27).
■ Die Elimination war bei den Copolymeren verringert.
■ V_{C} und kₑₗ zeigen keine erkennbare Dosisabhängigkeit.
■ Die berechnete AUC war für PEI 25 kDa und Beispiel 1 proportional zur Dosis, die Steigung der Geraden AUC/Dosis war bei Copolymer Beispiel 1 größer
■ Hauptorgane der Verteilung nach 120 Minuten waren Leber, Niere und Milz. Für die 6:1 Komplexe zeigen die Copolymere im Vergleich zu PEI 25 kDa eine verringerte Aufnahme in Leber und Milz, sowie eine höhere Aufnahme in die Niere.

## Patentansprüche

1. Verbindung der Formel I oder II
(I) A(-X-B)ₙ
(II) C(-Y-D)ₘ
in welcher
A für ein hydrophiles, nichtionisches, lineares oder verzweigtes Polymer mit einem Molekulargewicht von 100 bis 10.000.000 g/mol steht;
B für ein lineares oder verzweigtes Polyethylenimin (PEI) mit einem Molekulargewicht von 100 bis 1.000.000 g/mol steht;
X für eine direkte Bindung der Blöcke A und B oder für ein Brückenglied mit folgenden Strukturen steht, dessen C-teminale Seite mit einem Stickstoffatom des PEIs verknüpft ist:
-OC(O)NH(CH₂)ₒNHC(O)- mit o = 1 bis 20,
-OC(O)NH(Aryl)NHC(O)- mit Aryl = aromatische Einheit ,
-O(CH₂)ₚC(O)- mit p = 1 bis 10,
-OC(O)-, oder
-O(CH₂)_{q}- mit q = 1 bis 20;
n für eine ganze Zahl 1 bis 200 steht;
C für ein lineares oder verzweigtes PEI mit einem Molekulargewicht von 100 bis 1.000.000 g/mol;
D für einen Rest eines über O gebundenen Polyethylenglykols der Formel
-(CH₂CH₂O)_{n'}-R¹
steht, worin n' = 3 bis 25.000 ist und R¹ Wasserstoff, ein aliphatischer Rest oder eine andere OH-Schutzgruppe oder ein zellulärer Ligand ist;
Y für eine direkte Bindung der Blöcke C und D oder für ein Brückenglied mit folgenden Strukturen steht, dessen C-teminale Seite mit einem Stickstoffatom des PEIs verknüpft ist:
-C(O)NH(Aryl)NHC(O)O- mit Aryl = aromatische Einheit,
-(CH₂)ₜC(O)O- mit t = 2 bis 10,
-CH₂CH(OH)CH₂O-, oder
-(CH₂)ᵤO- mit u = 1 bis 20,
und
m für eine ganze Zahl von 1 bis 200 steht
mit der Maßgabe, daß die Reste und Variablen in Formel II so definiert sind, daß keine Verbindungen der Formel I davon umfaßt werden.

2. Verbindung gemäß Anspruch 1, in welcher
A für ein hydrophiles, nichtionisches, lineares oder verzweigtes Polymer mit einem Molekulargewicht von 1.000 bis 100.000 g/mol steht;
B für ein lineares oder verzweigtes Polyethylenimin (PEI) mit einem Molekulargewicht von 400 bis 100.000 g/mol steht;
X für eine direkte Bindung der Blöcke A und B oder für ein Brückenglied mit folgenden Strukturen steht, dessen C-teminale Seite mit einem Stickstoffatom des PEIs verknüpft ist:
-OC(O)NH(CH₂)ₒNHC(O)- mit o = 2 bis 10,
-OC(O)NH(Aryl)NHC(O)- mit Aryl = aromatische Einheit mit einem Kern,
-O(CH₂)ₚC(O)- mit p = 1 bis 3,
-OCH₂CH(OH)CH₂-,
-OC(O)-, oder
-O(CH₂)_{q}- mit q = 1 bis 6,
n für eine ganze Zahl von 1 bis 50,
C für ein lineares oder verzweigtes PEI mit einem Molekulargewicht von 400 bis 100.000 g/mol steht;
D für einen Rest eines über O gebundenen Polyethylenglykols der Formel
-(CH₂CH₂O)ₙ-R¹
steht, worin n' = 10 bis 5.000 ist und R¹ Wasserstoff, ein aliphatischer Rest oder eine andere OH-Schutzgruppe oder ein zellulärer Ligand ist;
Y für eine direkte Bindung der Blöcke C und D oder für ein Brückenglied mit folgenden Strukturen steht, dessen C-teminale Seite mit einem Stickstoffatom des PEls verknüpft ist:
-C(O)NH(Aryl)NHC(O)O- mit Aryl = aromatische Einheit mit einem Kern,
-(CH₂)ₜC(O)O- mit t = 2 bis 3,
-CH₂CH(OH)CH₂O-, oder
-(CH₂)ᵤO- mit u = 1 bis 6;
und
m für eine ganze Zahl von 1 bis 100 steht,
mit der Maßgabe, daß die Reste und Variablen in Formel II so definiert sind, daß keine Verbindungen der Formel I davon umfaßt werden.

3. Verbindung gemäß Anspruch 1 oder 2, in welcher
A für ein hydrophiles, nichtionisches, lineares oder verzweigtes Polymer mit einem Molekulargewicht von 5.000 bis 50.000 g/mol steht;
B für ein lineares oder verzweigtes Polyethylenimin (PEI) mit einem Molekulargewicht von 400 bis 50.000 g/mol steht;
X für eine direkte Bindung der Blöcke A und B oder für ein Brückenglied mit folgenden Strukturen steht, dessen C-teminale Seite mit einem Stickstoffatom des PEls verknüpft ist
-OC(O)NH(CH₂)ₒNHC(O)- mit o = 4 bis 6,
-OC(O)NH(Aryl)NHC(O)- mit Aryl = Toluyl-,
-O(CH₂)ₚC(O)- mit p = 1,
-OCH₂CH(OH)CH₂-,
-OC(O)-, oder
-O(CH₂)q- mit q = 1 bis 3;
n für eine ganze Zahl von 1 bis 12 steht;
C für ein lineares oder verzweigtes PEI mit einem Molekulargewicht von 400 bis 50.000 g/mol steht;
D für einen Rest eines über O gebundenen Polyethylenglykols der Formel
-(CH₂CH₂O)_{n'}-R¹
steht, worin n' = 10 bis 1.000 ist und R¹ Wasserstoff, ein aliphatischer Rest oder eine andere OH-Schutzgruppe oder ein zellulärer Ligand ist;
Y für eine direkte Bindung der Blöcke C und D oder für ein Brückenglied mit folgenden Strukturen steht, dessen C-teminale Seite mit einem Stickstoffatom des PEls verknüpft ist:
-C(O)NH(Aryl)NHC(O)O- mit Aryl = Toluyl-,
-(CH₂)ₜC(O)O- mit t = 2,
-CH₂CH(OH)CH₂O-, oder
-(CH₂)ᵤO- mit u = 1 bis 3;
und
m für eine ganze Zahl von 1 bis 50 steht
mit der Maßgabe, daß die Reste und Variablen in Formel II so definiert sind, daß keine Verbindungen der Formel I davon umfaßt werden.

4. Verbindung gemäß einem der Ansprüche 1 bis 3, welche die Formel I aufweist.

5. Verbindung gemäß einem der Ansprüche 1 bis 3, welche die Formel II aufweist

6. Verbindung gemäß einem der Ansprüche 1 bis 4, in welcher X für ein Brückenglied der Formel -OC(O)NH(CH₂)ₒNHC(O)- steht

7. Verwendung einer Verbindung der Formel II, in welcher Y für ein Brückenglied der Formel -C(O)NH(CH₂)ₛNHC(O)O- mit s = 1 - 10 steht und die übrigen Reste wie in einem der Ansprüche 1 bis 3 definiert sind, zur Komplexierung von Polynukleinsäuren in wäßrigen Systemen.

8. Verfahren zur Herstellung einer Verbindung der Formel I gemäß einem der Ansprüche 1 bis 4, **dadurch gekennzeichnet, daß** man
a) Verbindungen der allgemeinen Formel V
(V) A-(OH)ₙ
mit A und n = wie in Formel I
mit Diisocyanat umsetzt, oder
b) Verbindungen der allgemeinen Formel VI
(VI) A-(NH₂)ₙ
(mit A und n = wie in Formel I definiert)
bei der Polymerisation von Ethylenimin dem Reaktionsansatz von Beginn der Polyreaktion an oder erst später im Verlauf der Polyreaktion zufügt, oder
c) Verbindungen der allgemeinen Formel VII
(VII) A-(OS(O)₂R⁴)ₙ
mit A wie in Formel I und R⁴ = aliphatischer oder aromatischer Rest als Makroinitiator für die Polymerisation von Ethylenimin einsetzt.

9. Verfahren zur Herstellung von Verbindungen der Formel II gemäß einem der Ansprüche 1 bis 3 und 5, **dadurch gekennzeichnet, daß** man Verbindungen der allgemeinen Formel IX
(IX) D-OH
(mit D wie in Formel II definiert)
zunächst mit Diisocyanat und anschließend die erhaltene Verbindung mit linearem oder verzweigtem Polyethylenimin umsetzt.

10. Verwendung einer Verbindung der Formel 1 oder II
(I) A(-X-B)ₙ
(II) C(-Y-D)ₘ
in welcher
A für ein hydrophiles, nichtionisches, lineares oder verzweigtes Polymer mit einem Molekulargewicht von 100 bis 10.000.000 g/mol steht;
B für ein lineares oder verzweigtes Polyethylenimin (PEI) mit einem Molekulargewicht von 100 bis 1.000.000 g/mol steht;
X für eine direkte Bindung der Blöcke A und B oder für ein Brückenglied mit folgenden Strukturen steht, dessen C-teminale Seite mit einem Stickstoffatom des PEls verknüpft ist
-OC(O)NH(CH₂)ₒNHC(O)- mit o = 1 bis 20,
-OC(O)NH(Aryl)NHC(O)- mit Aryl = aromatische Einheit ,
-O(CH₂)ₚC(O)- mit p = 1 bis 10,
-OC(O)-, oder
-O(CH₂)_{q}- mit q =1 1 bis 20;
n für eine ganze Zahl 1 bis 200 steht;
C für ein lineares oder verzweigtes PEI mit einem Molekulargewicht von 100 bis 1.000.000 g/mol;
D für einen Rest eines über O gebundenen Polyethylenglykols der Formel
-(CH₂CH₂O)ₙ-R¹
steht, worin n' = 3 bis 25.000 ist und R¹ Wasserstoff, ein aliphatischer Rest oder eine andere OH-Schutzgruppe oder ein zellulärer Ligand ist;
Y für eine direkte Bindung der Blöcke C und D oder für ein Brückenglied mit folgenden Strukturen steht, dessen C-teminale Seite mit einem Stickstoffatom des PEIs verknüpft ist:
-C(O)NH(CH₂)ₛNHC(O)O- mit s = 1 bis 20,
-C(O)NH(Aryl)NHC(O)O- mit Aryl = aromatische Einheit,
-(CH₂)ₜC(O)O- mit t = 2 bis 10,
-CH₂CH(OH)CH₂O-, oder
-(CH₂)ᵤO- mit u = 1 bis 20,
und
m für eine ganze Zahl von 1 bis 200 steht,
mit der Maßgabe, daß die Reste und Variablen in Formel II so definiert sind, daß keine Verbindungen der Formel I davon umfaßt werden,
zur Komplexierung von Polynukleinsäuren in wäßrigen Systemen.

11. Verwendung gemäß Anspruch 10, **dadurch gekennzeichnet, daß** eine Verbindung der Formel I oder 11, worin
A für ein hydrophiles, nichtionisches, lineares oder verzweigtes Polymer mit einem Molekulargewicht von 1.000 bis 100.000 g/mol steht;
B für ein lineares oder verzweigtes Polyethylenimin (PEI) mit einem Molekulargewicht von 400 bis 100.000 g/mol steht;
X für eine direkte Bindung der Blöcke A und B oder für ein Brückenglied mit folgenden Strukturen steht, dessen C-teminale Seite mit einem Stickstoffatom des PEIs verknüpft ist:
-OC(O)NH(CH₂)ₒNHC(O)- mit o = 2 bis 10,
-OC(O)NH(Aryl)NHC(O)- mit Aryl = aromatische Einheit mit einem Kern,
-O(CH₂)ₚC(O)- mit p = 1 bis 3,
-OCH₂CH(OH)CH₂-,
-OC(O)-, oder
-O(CH₂)q- mit q = 1 bis 6,
n für eine ganze Zahl von 1 bis 50,
C für ein lineares oder verzweigtes PEI mit einem Molekulargewicht von 400 bis 100.000 g/mol steht;
D für einen Rest eines über O gebundenen Polyethylenglykols der Formel
-(CH₂CH₂O)ₙ-R¹
steht, worin n' = 10 bis 5.000 ist und R¹ Wasserstoff, ein aliphatischer Rest oder eine andere OH-Schutzgruppe oder ein zellulärer Ligand ist;
Y für eine direkte Bindung der Blöcke C und D oder für ein Brückenglied mit folgenden Strukturen steht, dessen C-teminale Seite mit einem Stickstoffatom des PEIs verknüpft ist:
-C(O)NH(CH₂)ₛNHC(O)O- mit s = 2 bis 10,
-C(O)NH(Aryl)NHC(O)O- mit Aryl = aromatische Einheit mit einem Kern,
-(CH₂)ₜC(O)O- mit t = 2 bis 3,
-CH₂CH(OH)CH₂O-, oder
-(CH₂)ᵤO- mit u = 1 bis 6;
und
m für eine ganze Zahl von 1 bis 100 steht,
mit der Maßgabe, daß die Reste und Variablen in Formel 11 so definiert sind, daß keine Verbindungen der Formel I davon umfaßt werden,
verwendet wird.

12. Verwendung gemäß einem der Ansprüche 10 oder 11, **dadurch gekennzeichnet, daß** eine Verbindung der Formel I oder II, worin
A für ein hydrophiles, nichtionisches, lineares oder verzweigtes Polymer mit einem Molekulargewicht von 5.000 bis 50.000 g/mol steht;
B für ein lineares oder verzweigtes Polyethylenimin (PEI) mit einem Molekulargewicht von 400 bis 50.000 g/mol steht;
X für eine direkte Bindung der Blöcke A und B oder für ein Brückenglied mit folgenden Strukturen steht, dessen C-teminale Seite mit einem Stickstoffatom des PEIs verknüpft ist
-OC(O)NH(CH₂)ₒNHC(O)- mit o = 4 bis 6,
-OC(O)NH(Aryl)NHC(O)- mit Aryl = Toluyl-,
-O(CH₂)ₚC(O)- mit p = 1,
-OCH₂CH(OH)CH₂-,
-OC(O)-, oder
-O(CH₂)_{q}- mit q = 1 bis 3;
n für eine ganze Zahl von 1 bis 12 steht;
C für ein lineares oder verzweigtes PEI mit einem Molekulargewicht von 400 bis 50.000 g/mol steht;
D für einen Rest eines über O gebundenen Polyethylenglykols der Formel
-(CH₂CH₂O)_{n'}-R¹
steht, worin n' = 10 bis 1.000 ist und R¹ Wasserstoff, ein aliphatischer Rest oder eine andere OH-Schutzgruppe oder ein zellulärer Ligand ist;
Y für eine direkte Bindung der Blöcke C und D oder für ein Brückenglied mit folgenden Strukturen steht, dessen C-teminale Seite mit einem Stickstoffatom des PEIs verknüpft ist:
-C(O)NH(CH₂)ₛNHC(O)O- mit s = 4 bis 6,
-C(O)NH(Aryl)NHC(O)O- mit Aryl = Toluyl-,
-(CH₂)_{C}C(O)O- mit t = 2,
-CH₂CH(OH)CH₂O-, oder
-(CH₂)ᵤO- mit u = 1 bis 3;
und
m für eine ganze Zahl von 1 bis 50 steht,
mit der Maßgabe, daß die Reste und Variablen in Formel II so definiert sind, daß keine Verbindungen der Formel I davon umfaßt werden,
verwendet wird.

13. Verwendung einer Verbindung gemäß einem der Ansprüche 10 bis 12 zur Komplexierung von DNA in wäßrigen Systemen.

14. Verwendung einer Verbindung gemäß einem der Ansprüche 10 bis 12 zur Komplexierung von RNA in wäßrigen Systemen.

15. Verwendung einer Verbindung gemäß einem der Ansprüche 10 bis 12 zur Komplexierung von Ribozymen in wäßrigen Systemen.

16. Zusammensetzung, die mindestens eine Nukleinsäure und eine Verbindung der Formel I oder II, die wie in einem der Ansprüche 10 bis 12 definiert ist, umfaßt.

17. Verwendung einer Verbindung der Formel I oder II, die wie in einem der Ansprüche 10 bis 12 definiert sind, als Tensid.

## Claims

1. A compound of the formula I or II
(I) A(-X-B)ₙ
(II) C(-Y-D)ₘ
in which
A is a hydrophilic, nonionic, linear or branched polymer with a molecular weight of from 100 to 10 000 000 g/mol;
B is a linear or branched polyethyleneimine (PEI) with a molecular weight of from 100 to 1000 000 g/mol;
X is a direct linkage of blocks A and B or a linker with the following structures whose C-terminal side is linked to a nitrogen atom of the PEI:
-OC(O)NH(CH₂)ₒNHC(O)- with o = 1 to 20,
-OC(O)NH(aryl)NHC(O)- with aryl = aromatic unit,
-O(CH₂)ₚC(O)- with p = 1 to 10,
-OC(O)-, or
-O(CH₂)_{q}- with q = 1 to 20;
n is an integer from 1 to 200;
C is a linear or branched PEI with a molecular weight of from 100 to 1 000 000 g/mol;
D is a residue of a polyethylene glycol of the formula
-(CH₂CH₂O)_{n'}-R¹
which is bonded via 0 and in which n' is from 3 to 25 000, and R¹ is hydrogen, an aliphatic residue or another OH-protective group or a cellular ligand;
Y is a direct linkage of blocks C and D or a linker with the following structures whose C-terminal side is linked to a nitrogen atom of the PEI:
-C(O)NH(aryl)NHC(O)O- with aryl = aromatic unit,
-(CH₂)ₜC(O)O- with t = 2 to 10,
-CH₂CH(OH)CH₂O-, or
-(CH₂)ᵤO- with u = 1 to 20,
and
m is an integer from 1 to 200,
with the proviso that the residues and variables in formula II are defined so that no compounds of the formula I are included thereby.

2. A compound as claimed in claim 1, in which
A is a hydrophilic, nonionic, linear or branched polymer with a molecular weight of from 1000 to 100 000 g/mol;
B is a linear or branched polyethyleneimine (PEI) with a molecular weight of from 400 to 100 000 g/mol;
X is a direct linkage of blocks A and B or a linker with the following structures whose C-terminal side is linked to a nitrogen atom of the PEI:
-OC(O)NH(CH₂)ₒNHC(O)- with 0 = 2 to 10,
-OC(O)NH(aryl)NHC(O)- with aryl = aromatic unit with one nucleus,
-O(CH₂)ₚC(O)- with p = 1 to 3,
-OCH₂CH(OH)CH₂-,
-OC(O)-, or
-O(CH₂)_{q}- with q = 1 to 6,
n is an integer from 1 to 50,
C is a linear or branched PEI with a molecular weight of from 400 to 100 000 g/mol;
D is a residue of a polyethylene glycol of the formula
-(CH₂CH₂O)ₙ,-R¹
which is bonded via O and in which n' is from 10 to 5000, and R¹ is hydrogen, an aliphatic residue or another OH-protective group or a cellular ligand;
Y is a direct linkage of blocks C and D or a linker with the following structures whose C-terminal side is linked to a nitrogen atom of the PEI:
-C(O)NH(aryl)NHC(O)O- with aryl = aromatic unit with one nucleus,
-(CH₂)ₜC(O)O- with t = 2 to 3,
-CH₂CH(OH)CH₂O-, or
-(CH₂)ᵤO- with u = 1 to 6;
and
m is an integer from 1 to 100,
with the proviso that the residues and variables in formula II are defined so that no compounds of the formula I are included thereby.

3. A compound as claimed in claim 1 or 2, in which
A is a hydrophilic, nonionic, linear or branched polymer with a molecular weight of from 5000 to 50 000 g/mol;
B is a linear or branched polyethyleneimine (PEI) with a molecular weight of from 400 to 50 000 g/mol;
X is a direct linkage of blocks A and B or a linker with the following structures whose C-terminal side is linked to a nitrogen atom of the PEI:
-OC(O)NH(CH₂)ₒNHC(O)- with 0 = 4 to 6,
-OC(O)NH(aryl)NHC(O)- with aryl = tolyl,
-O(CH₂)ₚC(O)- with p =1,
-OCH₂CH(OH)CH₂-,
-OC(O)-, or
-O(CH₂)q- with q = 1 to 3;
n is an integer from 1 to 12;
C is a linear or branched PEI with a molecular weight of from 400 to 50 000 g/mol;
D is a residue of a polyethylene glycol of the formula
-(CH₂CH₂O)ₙ-R¹
which is bonded via 0 and in which n' is from 10 to 1000, and R¹ is hydrogen, an aliphatic residue or another OH-protective group or a cellular ligand;
Y is a direct linkage of blocks C and D or a linker with the following structures whose C-terminal side is linked to a nitrogen atom of the PEI:
-C(O)NH(aryl)NHC(O)O- with aryl = tolyl,
-(CH₂)ₜC(O)O- with t = 2,
-CH₂CH(OH)CH₂O-, or
-(CH₂)ᵤO- with u = 1 to 3;
and
m is an integer from 1 to 50,
with the proviso that the residues and variables in formula II are defined so that no compounds of the formula I are included thereby.

4. A compound as claimed in any one of claims 1 to 3, which has formula I.

5. A compound as claimed in any one of claims 1 to 3, which has formula II.

6. A compound as claimed in any one of claims 1 to 4, in which X is a linker of the formula -OC(O)NH(CH₂)ₒNHC(O)-.

7. Use of a compound of the formula II in which Y is a linker of the formula -C(O)NH(CH₂)ₛNHC(O)O- with s = 1-10, and the other residues are as defined in any one of claims 1 to 3 for complexing polynucleic adds in aqueous systems.

8. A process for preparing a compound of the formula I as claimed in any one of claims 1 to 4, which comprises
a) reacting compounds of the general formula V
(V) A-(OH)ₙ with A and n = as in formula I
with diisocyanate, or
b) adding compounds of the general formula VI
(VI) A-(NH₂)ₙ (with A and n = as defined in formula I)
to the reaction mixture for the polymerization of ethyleneimine before the start of the polymerization or not until the polymerization is in progress, or
c) employing compounds of the general formula VII
(VII) A-(OS(O)₂R⁴)ₙ with A as in formula I and R⁴ = aliphatic or aromatic residue as macroinitiator for the polymerization of ethyleneimine.

9. A process for preparing compounds of the formula II as claimed in any one of claims 1 to 3 and 5, which comprises initially reacting compounds of the general formula IX
(IX) D-OH (with D as defined in formula II)
with diisocyanate and subsequently reading the resulting compound with linear or branched polyethyleneimine.

10. Use of a compound of the formula I or II
(I) A(-X-B)ₙ
(II) C(-Y-D)ₘ
in which
A is a hydrophilic, nonionic, linear or branched polymer with a molecular weight of from 100 to 10 000 000 g/mol;
B is a linear or branched polyethyleneimine (PEI) with a molecular weight of from 100 to 1000 000 g/mol;
X is a direct linkage of blocks A and B or a linker with the following structures whose C-terminal side is linked to a nitrogen atom of the PEI:
-OC(O)NH(CH₂)ₒNHC(O)- with 0 = 1 to 20,
-OC(O)NH(aryl)NHC(O)- with aryl = aromatic unit,
-O(CH₂)ₚC(O)- with p = 1 to 10,
-OC(O)-, or
-O(CH₂)_{q}- with q = 1 to 20;
n is an integer from 1 to 200;
C is a linear or branched PEI with a molecular weight of from 100 to 1 000 000 g/mol;
D is a residue of a polyethylene glycol of the formula
-(CH₂CH₂O)_{n'}-R¹
which is bonded via 0 and in which n' is from 3 to 25 000, and R¹ is hydrogen, an aliphatic residue or another OH-protective group or a cellular ligand;
Y is a direct linkage of blocks C and D or a linker with the following structures whose C-terminal is linked to a nitrogen atom of the PEI:
-C(O)NH(CH₂)ₛNHC(O)O- with s = 1 to 20,
-C(O)NH(aryl)NHC(O)O- with aryl = aromatic unit,
-(CH₂)ₜC(O)O- with t = 2 to 10,
-CH₂CH(OH)CH₂O-, or
-(CH₂)ᵤO- with u = 1 to 20,
and
m is an integer from 1 to 200,
with the proviso that the residues and variables in formula II are defined so that no compounds of the formula I are included thereby, for compelxing polynucleic acids in aqueous systems.

11. The use as claimed in claim 10, wherein a compound of the formula I or II, in which
A is a hydrophilic, nonionic, linear or branched polymer with a molecular weight of from 1000 to 100 000 g/mol;
B is a linear or branched polyethyleneimine (PEI) with a molecular weight of from 400 to 100 000 g/mol;
X is a direct linkage of blocks A and B or a linker with the following structures whose C-terminal side is linked to a nitrogen atom of the PEI:
-OC(O)NH(CH₂)ₒNHC(O)- with o = 2 to 10,
-OC(O)NH(aryl)NHC(O)- with aryl = aromatic unit with one nucleus,
-O(CH₂)ₚC(O)- with p = 1 to 3,
-OCH₂CH(OH)CH₂-,
-OC(O)-, or
-O(CH₂)_{q}- with q =1 to 6,
n is an integer from 1 to 50,
C is a linear or branched PEI with a molecular weight of from 400 to 100 000 g/mol;
D is a residue of a polyethylene glycol of the formula
-(CH₂CH₂O)_{n'}-R¹
which is bonded via 0 and in which n' is from 10 to 5000, and R¹ is hydrogen, an aliphatic residue or another OH-protective group or a cellular ligand;
Y is a direct linkage of blocks C and D or a linker with the following structures whose C-terminal side is linked to a nitrogen atom of the PEI:
-C(O)NH(CH₂)ₛNHC(O)O- with s = 2 to 10,
-C(O)NH(aryl)NHC(O)O- with aryl = aromatic unit with one nucleus,
-(CH₂)ₜC(O)O- with t = 2 to 3,
-CH₂CH(OH)CH₂O-, or
-(CH₂)ᵤO- with u = 1 to 6;
and
m is an integer from 1 to 100,
with the proviso that the residues and variables in formula II are defined so that no compounds of the formula I are included thereby,
is used.

12. The use as daimed in claim 10 or 11, wherein a compound of the formula I or II, in which
A is a hydrophilic, nonionic, linear or branched polymer with a molecular weight of from 5000 to 50 000 g/mol;
B is a linear or branched polyethyleneimine (PEI) with a molecular weight of from 400 to 50 000 g/mol;
X is a direct linkage of blocks A and B or a linker with the following structures whose C-terminal side is linked to a nitrogen atom of the PEI:
-OC(O)NH(CH₂)ₒNHC(O)- with o = 4 to 6,
-OC(O)NH(aryl)NHC(O)- with aryl = tolyl,
-O(CH₂)ₚC(O)- with p = 1,
-OCH₂CH(OH)CH₂-,
-OC(O)-, or
-O(CH₂)_{q} with q = 1 to 3;
n is an integer from 1 to 12;
C is a linear or branched PEI with a molecular weight of from 400 to 50 000 g/mol;
D is a residue of a polyethylene glycol of the formula
-(CH₂CH₂O)_{n'}-R¹
which is bonded via O and in which n' is from 10 to 1000, and R¹ is hydrogen, an aliphatic residue or another OH-protective group or a cellular ligand;
Y is a direct linkage of blocks C and D or a linker with the following structures whose C-terminal side is linked to a nitrogen atom of the PEI:
-C(O)NH(CH₂)ₛNHC(O)O- with s = 4 to 6,
-C(O)NH(aryl)NHC(O)O- with aryl = tolyl,
-(CH₂)ₜC(O)O- with t = 2,
-CH₂CH(OH)CH₂O-, or
-(CH₂)uO- with u =1 to 3;
and
m is an integer from 1 to 50,
with the proviso that the residues and variables in formula II are defined so that no compounds of the formula I are included thereby,
is used.

13. The use of a compound of any one of claims 10 to 12 for complexing DNA in aqueous systems.

14. The use of a compound of any one of claims 10 to 12 for complexing RNA. in aqueous systems.

15. The use of a compound of any one of claims 10 to 12 for complexing ribozymes in aqueous systems.

16. A compostion which comprises at least one nucleic acid and one compound of the formula I or II which is as defined in any one of daims 10 to 12.

17. Use of a compound of the formula I or II, according to any one of claims 10 to 12, as surfactant

## Revendications

1. Composé de la formule I ou II
(I) A(-X-B)ₙ
(II) C(-Y-D)ₘ
dont
A représente un polymère hydrophile non ionique, linéaire ou ramifié, avec un poids moléculaire de 100 à 10.000.000 g/mol;
B représente une polyéthylèneimine (PEI) linéaire ou ramifiée, avec un poids moléculaire de 100 à 1.000.000 g/mol;
X représente une liaison directe des blocs A et B ou un élément de raccord avec les structures suivantes, dont le côté C-teminal est lié à un atome d'azote de la PEI:
-OC(O)NH(CH₂)ₒNHC(O)- avec o = 1 à 20,
-OC(O)NH(aryle)NHC(O)- avec aryle = unité aromatique,
-O(CH₂)ₚC(O)- avec p = 1 à 10,
-OC(O)-, ou
-O(CH₂)q- avec q = 1 à 20,
n représente un nombre entier de 1 à 200;
C représente une PEI linéaire ou ramifiée avec un poids moléculaire de 100 à 1.000.000 g/mol;
D représente un reste d'un polyéthylène glycol lié par O de la formule
-(CH₂CH₂O)_{n'}-R¹
dont n' = 3 à 25.000 et R¹ représente de l'hydrogène, un reste aliphatique ou un autre groupe de protection pour OH, ou un ligand cellulaire;
Y représente une liaison directe des blocs C et D ou un élément de raccord avec les structures suivantes, dont le côté C-teminal est lié à un atome d'azote PEI:
-C(O)NH(aryle)NHC(O)O- avec aryle = unité aromatique,
-(CH₂)ₜC(O)O- avec t = 2 à 10,
-CH₂CH(OH)CH₂O-, ou
-(CH₂)ᵤO- avec u = 1 à 20,
et
m représente un nombre entier de 1 à 200,
les restes et les variables de la formule II devant être définis de telle manière qu'ils n'englobent aucun composé de la formule I.

2. Composé selon la revendication 1, dont
A représente un polymère hydrophile non ionique, linéaire ou ramifié, avec un poids moléculaire de 1.000 à 100.000 g/mol;
B représente une polyéthylèneimine (PEI) linéaire ou ramifiée, avec un poids moléculaire de 400 à 100.000 g/mol;
X représente une liaison directe des blocs A et B ou un élément de raccord avec les structures suivantes, dont le côté C-teminal est lié à un atome d'azote de la PEI:
-OC(O)NH(CH₂)ₒNHC(O)- avec o = 2 à 10,
-OC(O)NH(aryle)NHC(O)- avec aryle = unité aromatique avec un noyau,
-O(CH₂)ₚC(O)- avec p = 1 à 3,
-OCH₂CH(OH)CH₂-,
-OC(O)-, ou
-O(CH₂)q- avec q = 1 à 6,
n représente un nombre entier de 1 à 50,
C représente une PEI linéaire ou ramifiée avec un poids moléculaire de 400 bis 100.000 g/mol;
D représente un reste d'un polyéthylène glycol lié par O de la formule
-(CH₂CH₂O)_{n'}-R¹
dont n' = 10 à 5.000 et R¹ représente de l'hydrogène, un reste aliphatique ou un autre groupe de protection pour OH, ou un ligand cellulaire;
Y représente une liaison directe des blocs C et D ou un élément de raccord avec les structures suivantes, dont le côté C-teminal est lié à un atome d'azote de la PEI:
-C(O)NH(aryle)NHC(O)O- avec aryle = unité aromatique avec un noyau
-(CH₂)ₜC(O)O- avec t = 2 à 3,
-CH₂CH(OH)CH₂O-, ou
-(CH₂)ᵤO- avec u = 1 à 6,
et
m représente un nombre entier de 1 à 100,
les restes et les variables de la formule II devant être définis de telle manière qu'ils n'englobent aucun composé de la formule I.

3. Composé selon la revendication 1 ou 2, dont
A représente un polymère hydrophile non ionique, linéaire ou ramifié, avec un poids moléculaire de 5.000 à 50.000 g/mol;
B représente une polyéthylèneimine (PEI) linéaire ou ramifiée, avec un poids moléculaire de 400 à 50.000 g/mol;
X représente une liaison directe des blocs A et B ou un élément de raccord avec les structures suivantes, dont le côté C-teminal est lié à un atome d'azote de la PEI:
-OC(O)NH(CH₂)ₒNHC(O)- avec o = 4 à 6,
-OC(O)NH(aryle)NHC(O)- avec aryle = toluyle-,
-O(CH₂)ₚC(O)- avec p = 1,
-OCH₂CH(OH)CH₂-,
-OC(O)-, ou
-O(CH₂)_{q}- avec q = 1 à 3,
n représente un nombre entier de 1 à 12,
C représente une PEI linéaire ou ramifiée avec un poids moléculaire de 400 bis 50.000 g/mol;
D représente un reste d'un polyéthylène glycol lié par O de la formule
-(CH₂CH₂O)_{n'}-R¹
dont n' = 10 à 1.000 et R¹ représente de l'hydrogène, un reste aliphatique ou un autre groupe de protection pour OH, ou un ligand cellulaire;
Y représente une liaison directe des blocs C et D ou un élément de raccord avec les structures suivantes, dont le côté C-teminal est lié à un atome d'azote de la PEI:
-C(O)NH(aryle)NHC(O)O- avec aryle = toluyle-,
-(CH₂)ₜC(O)O- avec t = 2,
-CH₂CH(OH)CH₂O-, ou
-(CH₂)ᵤO- avec u = 1 à 3,
et
m représente un nombre entier de 1 à 50,
les restes et les variables de la formule II devant être définis de telle manière qu'ils n'englobent aucun composé de la formule I.

4. Composé selon l'une des revendications 1 à 3, qui utilise la formule I.

5. Composé selon l'une des revendications 1 à 3, qui utilise la formule II.

6. Composé selon l'une des revendications 1 à 4, dont X représente un élément de raccord de la formule -OC(O)NH(CH₂)ₒNHC(O)-.

7. Utilisation d'un composé de la formule II, dont Y représente un élément de raccord de la formule -C(O)NH(CH₂)ₛNHC(O)O- avec s = 1 - 10 et les autres restes étant définis comme dans l'une des revendications 1 à 3, pour la complexation d'acides polynucléiques dans des systèmes aqueux.

8. Procédé de fabrication d'un composé de la formule I selon l'une des revendications 1 à 4, **caractérisé par** le fait
a) de transformer des composés de la formule générale V
(V) A-(OH)ₙ avec A et n = comme dans la formule I
avec du diisocyanate, ou
b) de rajouter au mélange de réaction des composés de la formule générale VI
(VI) A-(NH₂)ₙ (avec A et n = comme définis dans la formule I)
lors de la polymérisation d'éthylèneimine, dès le début de la polyréaction ou plus tard pendant la polyréaction, ou
c) d'utiliser des composés de la formule générale VII
(VII) A-(OS(O)₂R⁴)ₙ avec A comme dans la formule I et R⁴ = reste aliphatique ou aromatique en tant que macroinitiateur pour la polymérisation d'éthylèneimines.

9. Procédé de fabrication des composés de la formule II selon l'une des revendications 1 à 3 et 5, **caractérisé par** le fait de transformer des composés de la formule générale IX
(IX) D-OH (avec D comme défini dans la formule II)
d'abord avec du diisocyanate, et puis de faire réagir le composé obtenu ensuite avec de la polyéthylèneimine linéaire ou ramifiée.

10. Utilisation d'un composé de la formule I ou II
(I) A(-X-B)ₙ
(II) C(-Y-D)ₘ
dont
A représente un polymère hydrophile non ionique, linéaire ou ramifié, avec un poids moléculaire de 100 à 10.000.000 g/mol;
B représente une polyéthylèneimine (PEI) linéaire ou ramifiée, avec un poids moléculaire de 100 à 1.000.000 g/mol;
X représente une liaison directe des blocs A et B ou un élément de raccord avec les structures suivantes, dont le côté C-teminal est lié à un atome d'azote de la PEI:
-OC(O)NH(CH₂)ₒNHC(O)- avec o = 1 à 20,
-OC(O)NH(aryle)NHC(O)- avec aryle = unité aromatique,
-O(CH₂)pC(O)- avec p = 1 à 10,
-OC(O)-, ou
-O(CH₂)_{q}- avec q = 1 à 20;
n représente un nombre entier de 1 à 200;
C représente une PEI linéaire ou ramifiée avec un poids moléculaire de 100 bis 1.000.000 g/mol;
D représente un reste d'un polyéthylène glycol lié par O de la formule
-(CH₂CH₂O)_{n'}-R¹
dont n' = 3 à 25.000 et R¹ représente de l'hydrogène, un reste aliphatique ou un autre groupe de protection pour OH, ou un ligand cellulaire;
Y représente une liaison directe des blocs C et D ou un élément de raccord avec les structures suivantes, dont le côté C-teminal est lié à un atome d'azote de la PEI:
-C(O)NH(CH₂)_{S}NHC(O)O- avec s = 1 à 20
-C(O)NH(aryle)NHC(O)O- avec aryle = unité aromatique,
-(CH₂)ₜC(O)O- avec t = 2 à 10,
-CH₂CH(OH)CH₂O-, ou
-(CH₂)ᵤO- avec u = 1 à 20,
et
m représente un nombre entier de 1 à 200,
les restes et les variables de la formule II devant être définis de telle manière qu'ils n'englobent aucun composé de la formule I,
pour la complexation d'acides polynucléiques dans des systèmes aqueux.

11. Utilisation selon la revendication 10, **caractérisée par** le fait d'utiliser un composé de la formule I ou II, dont
A représente un polymère hydrophile non ionique, linéaire ou ramifié, avec un poids moléculaire de 1.000 à 100.000 g/mol;
B représente une polyéthylèneimine (PEI) linéaire ou ramifiée, avec un poids moléculaire de 400 à 100.000 g/mol;
X représente une liaison directe des blocs A et B ou un élément de raccord avec les structures suivantes, dont le côté C-teminal est lié à un atome d'azote de la PEI:
-OC(O)NH(CH₂)ₒNHC(O)- avec o = 2 à 10,
-OC(O)NH(aryle)NHC(O)- avec aryle = unité aromatique avec un noyau
-O(CH₂)pC(O)- avec p = 1 à 3,
-OCH₂CH(OH)CH₂-
-OC(O)-, ou
-O(CH₂)_{q}- avec q = 1 à 6;
n représente un nombre entier de 1 à 50;
C représente une PEI linéaire ou ramifiée avec un poids moléculaire de 400 à 100.000 g/mol;
D représente un reste d'un polyéthylène glycol lié par O de la formule
-(CH₂CH₂O)_{n'}-R¹
dont n' = 10 à 5.000 et R¹ représente de l'hydrogène, un reste aliphatique ou un autre groupe de protection pour OH, ou un ligand cellulaire;
Y représente une liaison directe des blocs C et D ou un élément de raccord avec les structures suivantes, dont le côté C-teminal est lié à un atome d'azote de la PEI:
-C(O)NH(CH₂)ₛNHC(O)O- avec s = 2 à 10,
-C(O)NH(aryle)NHC(O)O- avec aryle = unité aromatique avec un noyau
-(CH₂)ₜC(O)O- avec t = 2 à 3,
-CH₂CH(OH)CH₂O-, ou
-(CH₂)ᵤO- avec u = 1 à 6,
et
m représente un nombre entier de 1 à 100,
les restes et les variables de la formule Il devant être définis de telle manière qu'ils n'englobent aucun composé de la formule 1.

12. Utilisation selon l'une des revendications 10 ou 11, **caractérisée par** le fait d'utiliser un composé de la formule I ou II, dont
A représente un polymère hydrophile non ionique, linéaire ou ramifié, avec un poids moléculaire de 5.000 à 50.000 g/mol;
B représente une polyéthylèneimine (PEI) linéaire ou ramifiée, avec un poids moléculaire de 400 à 50.000 g/mol;
X représente une liaison directe des blocs A et B ou un élément de raccord avec les structures suivantes, dont le côté C-teminal est lié à un atome d'azote de la PEI:
-OC(O)NH(CH₂)ₒNHC(O)- avec o = 4 à 6,
-OC(O)NH(aryle)NHC(O)- avec aryle = toluyle-,
-O(CH₂)ₚC(O)- avec p = 1,
-OCH₂CH(OH)CH₂-
-OC(O)-, ou
-O(CH₂)_{q}- avec q = 1 à 3;
n représente un nombre entier de 1 à 12;
C représente une PEI linéaire ou ramifiée avec un poids moléculaire de 400 bis 50.000 g/mol;
D représente un reste d'un polyéthylène glycol lié par O de la formule
-(CH₂CH₂O)_{n'}-R¹
dont n' = 10 à 1.000 et R¹ représente de l'hydrogène, un reste aliphatique ou un autre groupe de protection pour OH, ou un ligand cellulaire;
Y représente une liaison directe des blocs C et D ou un élément de raccord avec les structures suivantes, dont le côté C-teminal est lié à un atome d'azote de la PEI:
-C(O)NH(CH₂)_{S}NHC(O)O- avec s = 4 à 6,
-C(O)NH(aryle)NHC(O)O- avec aryle = toluyle-,
-(CH₂)ₜC(O)O- avec t = 2,
-CH₂CH(OH)CH₂O-, ou
-(CH₂)ᵤO- avec u = 1 à 3,
et
m représente un nombre entier de 1 à 50,
les restes et les variables de la formule II devant être définis de telle manière qu'ils n'englobent aucun composé de la formule I.

13. Utilisation d'un composé selon l'une des revendications 10 à 12, pour la complexation d'ADN dans des systèmes aqueux.

14. Utilisation d'un composé selon l'une des revendications 10 à 12, pour la complexation d'ARN dans des systèmes aqueux.

15. Utilisation d'un composé selon l'une des revendications 10 à 12, pour la complexation de ribozymes dans des systèmes aqueux.

16. Composition qui englobe au moins un acide nucléique et un composé de la formule I ou II, comme défini dans l'une des revendications 10 à 12.

17. Utilisation d'un composé de la formule I ou II, comme défini dans l'une des revendications 10 à 12, en tant que agent détersif.
